# EUROPEAN PATENT SPECIFICATION

(11) **EP 1 387 698 B1**
(45) Date of publication and mention of the grant of the patent: **14.11.2007**
(21) Application number: 02769112.0
(22) Date of filing: 06.05.2002
(51) Int. Cl.: A61K 39/395

(54) **METHOD OF PREVENTING CELL DEATH USING ANTIBODIES TO NEURAL THREAD PROTEINS**
VERFAHREN ZUR VORBEUGUNG VON ZELLTOD UNTER VERWENDUNG VON ANTIKÖRPERN GEGEN NEURAL THREAD PROTEINE
PROCEDE POUR PREVENIR LA MORT CELLULAIRE A L'AIDE D'ANTICORPS AGISSANT A L'ENCONTRE DE PROTEINES FILAMENTEUSES NERVEUSES

(30) Priority: 04.05.2001 US 288463 P
(43) Date of publication of application: 11.02.2004
(73) Proprietor: NYMOX CORPORATION, Saint-Laurent,Quebec H4M 2V2 (CA)
(72) Inventor: AVERBACK, Paul, A., Beaconsfield, Québec H9W 4J1 (CA)
(74) Representative: Baldock, Sharon Claire
(86) International application number: PCT/CA2002/000681
(87) International publication number: WO 2002/089841

(56) References cited:
- WO-A-98/38204
- WO-A-99/00150
- WO-A-02/099036
- US-A- 5 948 634
- MONTE DE LA S M ET AL: "ALZHEIMER-ASSOCIATED NEURONAL THREAD PROTEIN-INDUCED APOPTOSIS AND IMPAIRED MITOCHONDRIAL FUNCTION IN HUMAN CENTRAL NERVOUS SYSTEM-DERIVED NEURONAL CELLS" JOURNAL OF NEUROPATHOLOGY AND EXPERIMENTAL NEUROLOGY, NEW YORK, NY, US, vol. 60, no. 2, February 2001 (2001-02), pages 195-207, XP009002171 ISSN: 0022-3069
- GOLUBNITSCHAJA-LABUDOVA O ET AL: "ALTERED GENE EXPRESSION IN LYMPHOCYTES OF PATIENTS WITH NORMAL-TENSION GLAUCOMA" CURRENT EYE RESEARCH, IRL PRESS, OXFORD, GB, vol. 21, no. 5, November 2000 (2000-11), pages 867-876, XP009002181 ISSN: 0271-3683
- XIAO-BIAO L ET AL: "BRAIN DELIVERY OF BIOTINYLATED NGF BOUNDED TO AN AVIDIN-TRANSFERRIN CONJUGATE" JOURNAL OF NATURAL TOXINS, COLLINS, CO, US, vol. 1, no. 9, February 2000 (2000-02), pages 73-83, XP008005585 ISSN: 1058-8108

### SEQUENCE LISTING

<110> NYMOX CORPORATION
   AVERBACK, Paul
<120> METHOD OF PREVENTING CELL DEATH USING ANTIBODIES TO NEURAL THREAD PROTEINS
<130> 10107-35
<140> PCT/CA02/00681
   <141> 2002-05-06
<150> US 60/288,463
   <151> 2001-05-04
<160> 8
<170> PatentIn Ver. 2.1
<210> 1
   <211> 122
   <212> PRT
   <213> Homo sapiens
<400> 1
   Met Met Val Cys Trp Asn Arg Phe Gly Lys Trp Val Tyr Phe Ile Ser
    1               5                  10                  15
   Ala Ile Phe Asn Phe Gly Pro Arg Tyr Leu Tyr His Gly Val Pro Phe
                20                  25                  30
   Tyr Phe Leu Ile Leu Val Arg Ile Ile Ser Phe Leu Ile Gly Asp Met
            35                  40                  45
   Glu Asp Val Leu Leu Asn Cys Thr Leu Leu Lys Arg Ser Ser Arg Phe
        50                  55                  60
   Arg Phe Trp Gly Ala Leu Val Cys Ser Met Asp Ser Cys Arg Phe Ser
    65                  70                  75                  80
   Arg Val Ala Val Thr Tyr Arg Phe Ile Thr Leu Leu Asn Ile Pro Ser
                    85                  90                  95
   Pro Ala Val Trp Met Ala Arg Asn Thr Ile Asp Gln Gln Val Leu Ser
               100                 105                 110
   Arg Ile Lys Leu Glu Ile Lys Arg Cys Leu
           115                 120
<210> 2
   <211> 112
   <212> PRT
   <213> Homo sapiens
<400> 2
<210> 3
   <211> 106
   <212> PRT
   <213> Homo sapiens
<400> 3
   Met Trp Thr Leu Lys Ser Ser Leu Val Leu Leu Leu Cys Leu Thr Cys
   1               5                   10                  15
   Ser Tyr Ala Phe Met Phe Ser Ser Leu Arg Gln Lys Thr Ser Glu Pro
               20                  25                  30
   Gln Gly Lys Val Pro Cys Gly Glu His Phe Arg Ile Arg Gln Asn Leu
           35                  40                  45
   Pro Glu His Thr Gln Gly Trp Leu Gly Ser Lys Trp Leu Trp Leu Leu
       50                  55                  60
   Phe Ala Val Val Pro Phe Val Ile Leu Lys Cys Gln Arg Asp Ser Glu
   65                  70                  75                  80
   Lys Asn Lys Val Arg Met Ala Pro Phe Phe Leu His His Ile Asp Ser
                   85                  90                  95
   Ile Ser Gly Val Ser Gly Lys Arg Met Phe
               100                 105
<210> 4
   <211> 106
   <212> PRT
   <213> Homo sapiens
<400> 4
   Met Phe Phe Val Leu Tyr Arg Phe Cys Phe Cys Phe Phe Glu Thr Glu
   1               5                   10                  15
   Ser His Ser Leu Thr Gln Ala Gly Val Gln Trp Cys Glu Leu Gly Ser
               20                  25                  30
   Pro Gln Pro Leu Pro Ser Gly Phe Lys Arg Phe Ser Cys Leu Ser Leu
           35                  40                  45
   Leu Ser Ser Trp Asp Tyr Ser His Glu Pro Pro His Pro Val Ile Cys
       50                  55                  60
   Ser Phe Leu Met Glu Lys Cys Leu Ile Leu Tyr Lys Pro Asn Gly Asp
   65                  70                  75                  80
   Thr Ile Gly Pro Ile Leu Val Gln Gln Gly Lys Arg Gln Lys Leu Tyr
                   85                  90                  95
   Ile Ser Ala Asp Leu Val His Leu Ile Ala
               100                 105
<210> 5
   <211> 98
   <212> PRT
   <213> Homo sapiens
<400> 5
   Glu Ala Tyr Tyr Thr Met Leu His Leu Pro Thr Thr Asn Arg Pro Lys
   1               5                   10                  15
   Ile Ala His Cys Ile Leu Phe Asn Gln Pro His Ser Pro Arg Ser Asn
               20                  25                  30
   Ser His Ser His Pro Asn Pro Leu Lys Leu His Arg Arg Ser His Ser
           35                  40                  45
   His Asn Arg Pro Arg Ala Tyr Ile Leu Ile Thr Ile Leu Pro Ser Lys
       50                  55                  60
   Leu Lys Leu Arg Thr His Ser Gln Ser His His Asn Pro Leu Ser Arg
   65                  70                  75                  80
   Thr Ser Asn Ser Thr Pro Thr Asn Ser Phe Leu Met Thr Ser Ser Lys
                   85                  90                  95
   Pro Arg
<210> 6
   <211> 75
   <212> PRT
   <213> Homo sapiens
<400> 6
   Ser Ser Ser Leu Gly Leu Pro Lys Cys Trp Asp Tyr Arg His Glu Leu
    1               5                  10                  15
   Leu Ser Leu Ala Leu Met Ile Asn Phe Arg Val Met Ala Cys Thr Phe
               20                  25                  30
   Lys Gln His Ile Glu Leu Arg Gln Lys Ile Ser Ile Val Pro Arg Lys
           35                  40                  45
   Leu Cys Cys Met Gly Pro Val Cys Pro Val Lys Ile Ala Leu Leu Thr
       50                  55                  60
   Ile Asn Gly His Cys Thr Trp Leu Pro Ala Ser
   65                  70                  75
<210> 7
   <211> 68
   <212> PRT
   <213> Homo sapiens
<400> 7
   Met Phe Val Phe Cys Leu Ile Leu Asn Arg Glu Lys Ile Lys Gly Gly
    1               5                  10                  15
   Asn Ser Ser Phe Phe Leu Leu Ser Phe Phe Phe Ser Phe Gln Asn Cys
               20                  25                  30
   Cys Gln Cys Phe Gln Cys Arg Thr Thr Glu Gly Tyr Ala Val Glu Cys
           35                  40                  45
   Phe Tyr Cys Leu Val Asp Lys Ala Ala Phe Glu Cys Trp Trp Phe Tyr
       50                  55                  60
   Ser Phe Asp Thr
   65
<210> 8
   <211> 61
   <212> PRT
   <213> Homo sapiens
<400> 8
   Met Glu Pro His Thr Val Ala Gln Ala Gly Val Pro Gln His Asp Leu
    1               5                  10                  15
   Gly Ser Leu Gln Ser Leu Leu Pro Arg Phe Lys Arg Phe Ser Cys Leu
               20                  25                  30
   Ile Leu Pro Lys Ile Trp Asp Tyr Arg Asn Met Asn Thr Ala Leu Ile
           35                  40                  45
   Lys Arg Asn Arg Tyr Thr Pro Glu Thr Gly Arg Lys Ser
       50                  55                  60

## Description

### BACKGROUND OF THE INVENTION

### 1. Field of the Invention

The present invention relates to use at least one antibody, antibody fragment or antibody derivative that recognises NTP for the manufacture of an agent for use in preventing or inhibiting cell death, and for use in treating conditions that require prevention, inhibition, and/or amelioration of cell death and tissue necrosis. The invention encompasses use of an antibody or antibodies, antibody derivative(s) or antibody fragment(s) that bind to neural thread proteins (NTP) for the manufacture of an agent for administration to a mammal experiencing cell death as a result of the presence of NTP. The antibody, antibody derivative or antibody fragment can be administered intramuscularly, orally, intravenously, intraperitoneally, intracerebrally (intraparenchymally), intracerebroventricularly, intratumorally, intralesionally, intradermally, intrathecally, intranasally, intraocularly, intraarterially, topically, transdermally, via an aerosol, infusion, bolus injection, implantation device, sustained release system etc., either alone or conjugated to a carrier.

### 2. Description of Related Art

AIzheimer's disease (AD) is a complex neurodegenerative disorder characterized by progressive impairments in memory, behavior, language, and visuo-spatial skills, ending ultimately in death. Hallmark pathologies within vulnerable regions include extracellular β-amyloid deposits, intracellular neurofibrillary tangles, synaptic loss, and extensive neuronal cell death. Research on the causes and treatments of Alzheimer's disease has led investigators down numerous avenues. Considerable evidence has implicated alterations in production or processing of the human amyloid precursor protein (APP) in the etiology of the disease. However, intensive research has proven that AD is a multifactorial disease with many different, perhaps overlapping, etiologies.

Because of this, those in the field have conducted significant research and clinical investigations to study the structural deficiencies, chemical changes, and functional abnormalities both within the brain and within different populations of nerve cells. The depth of such investigations and studies are represented by the following publications, which represent only a handful of the vast reports in this arena: Neurobiology of Alzheimer's Disease (D. Dawbarn and S. J. Allen, Editors), Bios, Oxford 1995; Dementia, (J. Whitehouse, Ed.), F.A. Davis Company, Philadelphia, 1993; Alzheimer's Disease: Senile Dementia and Related Disorders (Katzman, R, and R. L. Bick, Eds), Raven Press, New York, 1994, pages 47-51; Alzheimer's Disease and Related Disorders, Etiology, Pathogenesis and Therapeutics (Iqbol, K., et al., Eds.), Wiley, Chichester, 1999; Alzheimer's Disease: Advances in Clinical and Basic Research (Corain, B, Ed.), Wiley, New York, 1993; Alzheimer's Disease: Clinical and Treatment Perspectives (Cutler, N.R, et al., Eds.), Wiley, Chichester, 1995; Alzheimer's Disease: Therapeutic Strategies (Giacobini, E., Becker, R., Eds.), Birkhauser, Boston, 1994; Paykel, et al., Arch. Gen. Psychiat., 51:325-332 (1994); Amaducci, et al., Neurology, 36:922-931 (1986); McKhann, et al., Neurology 34:939-944 (1984), Heston et al., Arch. Gen. Psychiatry 38:1085-1090 (1981); Aging of the Brain (Gispen and Traber, editors), Elsevier Science Publishers, Amsterdam, 1983, pages 275-282; Heyman et al., Ann. Neurol 15:335-341 (1984); Brayne C. and P. Calloway, Lancet 1:1265-1267 (1988); Roth et al., Br. J. Psychiatry 149:698-709 (1986); Medical Research Council, Report from the NRC Alzheimer's Disease Workshop, London, England, 1987; Morris et al., Neurology 41:469-478 (1991); and the references cited within each of these publications.

To date, Alzheimer's disease is the third most expensive disease in the United States, costing society approximately $100 billion each year. It is one of the most prevalent illnesses in the elderly population, and with the aging of society, will become even more significant. Costs associated with AD include direct medical costs such as nursing home care, direct nonmedical costs such as in-home day care, and indirect costs such as lost patient and care giver productivity. Medical treatment and behavior modification may have economic benefits by slowing the rate of cognitive decline, delaying institutionalization, reducing care giver hours, and improving quality of life. Pharmacoeconomic evaluations have shown positive results regarding the effect of drug therapy and behavior modification on nursing home placement, cognition, and care giver time.

Neural thread proteins (NTP) are a family of recently characterized brain proteins. One member of this family, AD7C-NTP, is a ~41 kD membrane associated phosphoprotein with functions related to neuritic sprouting (de la Monte et al., J. Clin. Invest., 100:3093-3104 (1997); de la Monte et al., Alz. Rep., 2:327-332 (1999); de la Monte SM and Wands JR, Journal of Alzheimer's Disease, 3:345-353 (2001)). The gene that encodes AD7C-NTP and predicted protein sequence for AD7C-NTP has been identified and described (de la Monte et al., J. Clin. Invest., 100:3093-3104 (1997)). In addition to the ~41 kD species, other species of neural thread protein (~26 kD, ~21 kD, ~17 kD, and ~15 kD) have been identified and associated with neuroectodermal tumors, astrocytomas, and glioblastomas and with injury due to hypoxia, schema, or cerebral infarction (Xu et al., Cancer Research, 53:3823-3829 (1993); de la Monte et al., J. Neuropathol. Exp. Neurol., 55(10):1038-50 (1996), de la Monte et al., J. Neurol. Sci., 138(1-2):26-35 (1996); de la Monte et al., J. Neurol. Sci., 135(2):118-25 (1996); de la Monte et al., J. Clin. Invest., 100:3093-3104 (1997); and de la Monte et al., Alz. Rep., 2:327-332 (1999)).

Species of neural thread protein have been described and claimed in U.S. Patent Nos. 5,948,634; 5,948,888; and 5,830,670, all for "Neural Thread Protein Gene Expression and Detection of Alzheimer's Disease" and in U.S. Patent No. 6,071, 705 for "Method of Detecting Neurological Disease or Dysfunction." As described therein, NTP is upregulated and produced during cell death. Thus, dead and dying nerve cells are described as overproducing NTP, and accordingly, its presence indicates the death of nerve cells and the onset of Alzheimer's disease (AD).

Other species of neural thread protein have been identified as other products of the AD7c-NTP gene (e.g. a 112 amino acid protein described in NCBI Entrez-Protein database Accession #XP_032307 PID g15928971) or as being similar to neural thread proteins (e.g. a 106 amino acid protein described in NCBI Entrez-Protein database Accession #AAH14951 PID g15928971, another 106 amino acid protein described in NCBI Entrez-Protein database Accession #XP_039102 PID g18599339 and a 61 amino acid protein described in NCBI Entrez-Protein database Accession #AAH02534 PID g12803421).

There is compelling evidence linking the AD7C-NTP specie of neural thread protein in particular with AD and it is upregulated during cell death in AD. AD7C-NTP mRNA is upregulated in AD brain compared to controls; AD7C-NTP protein levels in brain and in CSF are higher in AD than controls; and AD7C-NTP immunoreactivity is found in senile plaques, in neurofibrillary tangles (NFT), in degenerating neurons, neuropil threads, and dystrophic neurotic sprouts in AD and Down syndrome brains (Ozturk et al., Proc. Natl. Acad. Sci, USA, 86:419-423 (1989); de la Monte et al., J. Clin. Invest., 86(3):1004-13 (1990); de la Monte et al., J. Neural. Sci., 113(2):152-64 (1992); de la Monte et al., Ann. Neurol., 32(6):733-42 (1992); de la Monte et al., J. Neuropathol. Exp. Neurol., 55(10):1035-50 (1996), de la Monte et al., J. Neurol. Sci., 138(1-2):26-35 (1996); de la Monte et al., J. Neurol. Sci., 135(2):118-25 (1996); de la Monte et al., J. Clin. Invest., 100:3093-3104 (1997); and de la Monte et al., Alz.. Rep., 2:327-332 (1999)). Immunocytochemistry demonstrated that the AD7C-NTP protein is localized within cells, within fine processes within the neuropil, or is extracellular in both AD and Down's Syndrome brains. de la Monte et al., Ann. Neurol., 32(6):733-42 (1992). Two types of cells contain NTP: neurons and astrocytes (*Id.*). The affected neurons are the large pyramidal type that typically contain the neurofibrillary tangles well known in AD brain (*Id.*).

Elevated levels of AD7C-NTP protein have been found in both CSF and urine of AD patients, showing its accuracy as a biochemical marker for this devastating illness (de la Monte and Wands, Front Biosci 7: 989-96 (2002); de la Monte and Wands, Journal of Alzheimer's Disease 3: 345-353 (2001); Munzar et al, Alzheimer's Reports 4: 61-65 (2001): Kahle et al, Neurology 54: 1498-1504 (2000) and Averback Neurology 55: 1068 (2000); Munzar et al, Alzheimer Reports 3: 155-159 (2000); de la Monte et al, Alzheimer's Reports 2: 327-332 (1999): Ghanbari et al, J Clin Lab Anal 12: 285-288 (1998); Ghanbari et al, J Clin Lab Anal 12: 223-226 (1998); Ghanbari et al, Journal of Contemporary Neurology 1998; 4A: 2-6 (1998); and de la Monte et al, J Clin Invest 100: 3093-3104 (1997).

Over-expression of the AD7C-NTP gene also has been linked to the process of cell death in Alzheimer's disease (de la Monte and Wands, J. Neuropatho. and Exp. Neuro., 60:195-207 (2001); de la Monte and Wands, Cell Mol Life Sci 58: 844-49 (2001). AD7C-NTP has also been identified in Down's Syndrome brain tissue (Wands et al., International Patent Publication No. WO 90,06993; de la Monte et al, J Neurol Sci 135: 118-25 (1996); de la Monte et al., Alz., Rep., 2:327-332 (1999)). There is some evidence that over-expression of the AD7c-NTP gene may also be associated with glaucoma (Golubnitschaja-Labudova et al, Curr Eye Res 21: 867-76 (2000)).

The present inventor recently discovered that released AD7C-NTP protein was cytotoxic and capable of causing cell death to other cells in tissue (as compared with up-regulated AD7C-NTP produced by the dying cell itself) Accordingly, it would be desirable to prevent, inhibit, modulate or ameliorate cell death and tissue necrosis associated with neural thread proteins, especially in AD brain.

### SUMMARY OF THE INVENTION

There exists a need to develop a method of preventing, inhibiting, modulating and/or ameliorating cell death and tissue necrosis in live tissue containing NTP. In particular, there exists a need to develop a method capable of preventing, inhibiting and/or ameliorating cell death and/or tissue necrosis in live tissue containing NTP in the brain. There also exists a need to develop a method of treating conditions caused by cell death and/or tissue necrosis due to the presence ofNTP in live tissue. There also exists a need to develop a method of treating cerebral amyloidosis by preventing, inhibiting and/or ameliorating cell death and/or tissue necrosis in live mammalian brain tissue containing NTP. There also exists a need to develop a method of controlling, inhibiting, modulating or ameliorating cell death and tissue necrosis in live tissue caused by NTP administered in order to remove or destroy harmful or unwanted tissue or cellular elements such as benign or malignant tumors in humans.

It is therefore a feature of an embodiment of the invention to provide use of at least are that recognises NTP for the manufacture of an agent for preventing, inhibiting and/or ameliorating cell death and/or tissue necrosis in live tissue containing NTP by contacting the live tissue containing NTP with at least one antibody that recognizes or binds to NTP, whereby the antibody is present in an amount sufficient to prevent, inhibit, reduce, control and/or ameliorate cell death and/or tissue necrosis caused by the presence of NTP.

In accordance with another feature of an embodiment of the invention, there is provided use of at least one antibody fragment that recognizes NTP for the manufacture of an agent for preventing, inhibiting and/or ameliorating cell death preventing, inhibiting and/or ameliorating cell death and/or tissue necrosis in live tissue containing NTP by contacting the live tissue containing NTP with an antibody fragment that recognizes or binds to NTP. The antibody fragment is present in an amount sufficient to prevent, inhibit, reduce, control and/or ameliorate cell death and/of tissue necrosis caused by the presence of NTP.

In accordance with another feature of an embodiment of the invention, there is provided use of at least are antibody derivative that recognizes NTP for the manufacture of an agent for preventing, inhibiting and/or ameliorating cell death and/or tissue necrosis in live tissue containing NTP by contacting the live tissue containing NTP with an antibody derivative that recognizes or binds to NTP. The antibody derivative is present in an amount sufficient to prevent, inhibit, reduce, control and/or ameliorate cell death and/or tissue necrosis caused by the presence of NTP.

In accordance with another feature of an embodiment of the invention, there is provided a composition for use in preventing and/or inhibiting cell death and/or tissue necrosis in live mammalian brain tissue containing NTP by contacting the live mammalian brain tissue containing NTP with a component containing at least an antibody, antibody derivative or antibody fragment recognizing or binding to NTP. The component is capable of crossing the blood-brain barrier.

In accordance with yet another feature of an embodiment of the invention, there is provided use of at least one antibody fragment or antibody derivative that recognises NTP for the manufacture of an agent for treating conditions caused by cell death and/or tissue necrosis due to the presence of NTP by contacting live tissue containing NTP with an antibody, antibody derivative or antibody fragment that recognizes or binds to NTP. The antibody, antibody derivative or antibody fragment is present in an amount sufficient to prevent, inhibit, reduce, and/or ameliorate cell death and/or tissue necrosis caused by the presence of NTP. In accordance with this use, an antibody or antibody fragment that recognizes or binds to NTP used to manufacture an agent to be administered to a mammal having a condition caused by cell death and/or tissue necrosis due to the presence of NTP in an amount sufficient to prevent, inhibit, reduce, and/or ameliorate cell death and/or tissue necrosis caused by the presence of NTP.

In accordance with another feature of an embodiment of the invention, there is provided a composition comprising an antibody, antibody derivative or antibody fragment that recognizes or binds to NTP, and a component that enables the antibody or antibody fragment to cross the blood-brain barrier.

In accordance with another feature of an embodiment of the invention, the composition is for use in treating cerebral amyloidois (by contacting live mammalian brain tissue containing NTP with a component containing at least an antibody, antibody derivative or antibody fragment recognizing or binding to NTP. The component is capable of crossing the blood-brain barrier).

In accordance with another feature of an embodiment of the invention, there is provided use of at least one antibody antibody fragment or antibody derivative that recognises NTP in the manufacture of a medicament for treating glaucoma caused by cell death and/or tissue caused by cell death and/or tissue necrosis due to the presence ofNTP by contacting the tissue containing NTP with at least an antibody, antibody derivative or antibody fragment recognizing or binding to NTP.

Also described is a method of treating conditions caused by cell death and/or tissue necrosis due to the presence ofNTP comprising administering a gene to a mammal in need thereof, whereby the gene expresses an antibody, antibody derivative or antibody fragment that recognizes or binds to NTP, and whereby the administration results in the antibody, antibody derivative or antibody fragment connoting live tissue containing NTP. The gene is administered in such a fashion that the antibody, antibody derivative or antibody fragment is present in an amount sufficient to prevent, inhibit and/or ameliorate cell death and/or tissue necrosis caused by the presence of NTP.

Also described is a method oftreating conditions caused by cell death and/or tissue necrosis due to the presence of NTP comprising administering a vaccine to a mammal in need thereof, whereby the vaccine induces the mammal to express or otherwise produce an antibody or antibody fragment that recognizes or binds to NTP, and whereby the administration results in the antibody or antibody fragment contacting live tissue containing NTP. The vaccine is administered in such a fashion that the antibody or antibody fragment is present in an amount sufficient to prevent, inhibit and/or ameliorate cell death and/or tissue necrosis caused by the presence of NTP.

Also described is a method of treating conditions caused by cell death and/or tissue necrosis due to the presence ofNTP comprising introducing or administering to or implanting in a mammal in need thereof cells, bacteria or viruses that are capable of expressing the antibody, antibody derivative or antibody fragment *in vivo,* whereby the cells, bacteria or viruses express an antibody, antibody derivative or antibody fragment that recognizes or binds to NTP, and whereby the administration results in the antibody, antibody derivative or antibody fragment contacting live tissue containing NTP. The cells, bacteria or viruses are introduced, administered or implanted in such a fashion and in such a quantity that the antibody, antibody derivative or antibody fragment is present in an amount sufficient to prevent, inhibit and/or ameliorate cell death and/or tissue necrosis caused by the presence of NTP.

These and other features of the invention will be readily apparent to those skilled in the art upon reading the detailed description that follows. It should be understood, however, that the detailed description and the specific examples, while indicating preferred embodiments of the invention are given by way of illustration only.

### BRIEF DESCRIPTION OF THE DRAWINGS

FIGs-1-4 are microphotographs of histopathological lesions induced by injections of NTP.
Figure 5 shows the complete amino acid sequences of the 122 amino acid neural thread protein (Sequence 40 from U.S. Patent No. 5,948,634; NCBI Entrez-Protein Accession #AAE25447).
Figure 6 shows the complete amino acid sequences of the 112 amino acid neural thread protein (NCBI Entrez-Protein Accession #XP_032307).
Figure 7 shows the 106 amino acid neural thread protein listed in NCBI Entrez-Protein Accession # AAH14951 PID g15928971.
Figure 8 shows the 106 amino acid neural thread protein listed in NCBI Entrez-Protein Accession # XP_039102, PID g18599339.
Figure 9 shows the complete amino acid sequences of the 98 amino acid neural thread protein (Sequence 30 from U.S. Patent No. 5,830,670; NCBI Entrez-Protein Accession #AAE13612).
Figure 10 shows the complete amino acid sequences of the 75 amino acid neural thread protein (Sequence 48 from U.S. Patent No. 5,948,634; NCBI Entrez-Protein Accession #AAE25448).
Figure 11 shows the complete amino acid sequences of the 68 amino acid neural thread protein (Sequence 36 from U.S. Patent No. 5,948,634; NCBI Entrez-Protein Accession #AAE25446).
Figure 12 shows the complete amino acid sequences of the 61 amino acid neural thread protein-like protein (NCBI Entrez-Protein Accession #AAH02534).

### DETAILED DESCRIPTION OF PREFERRED EMBODIMENTS

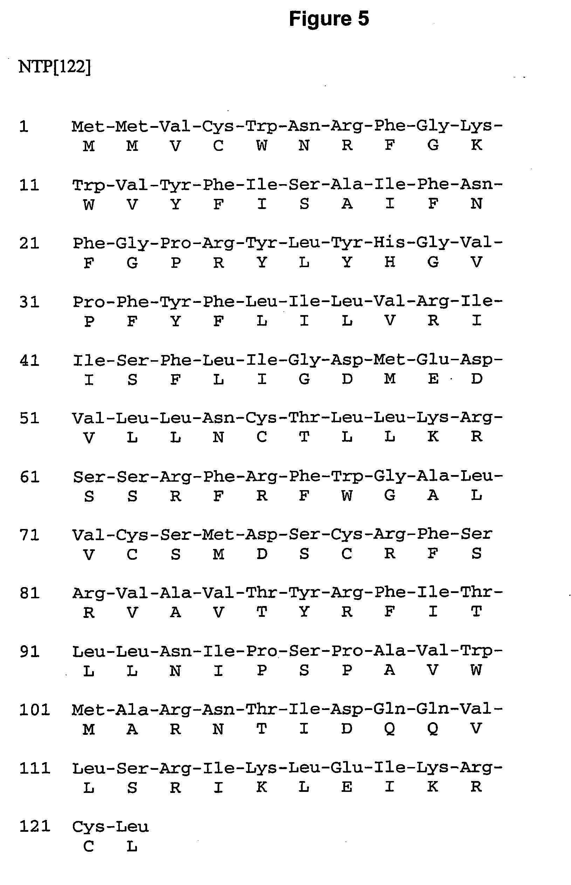

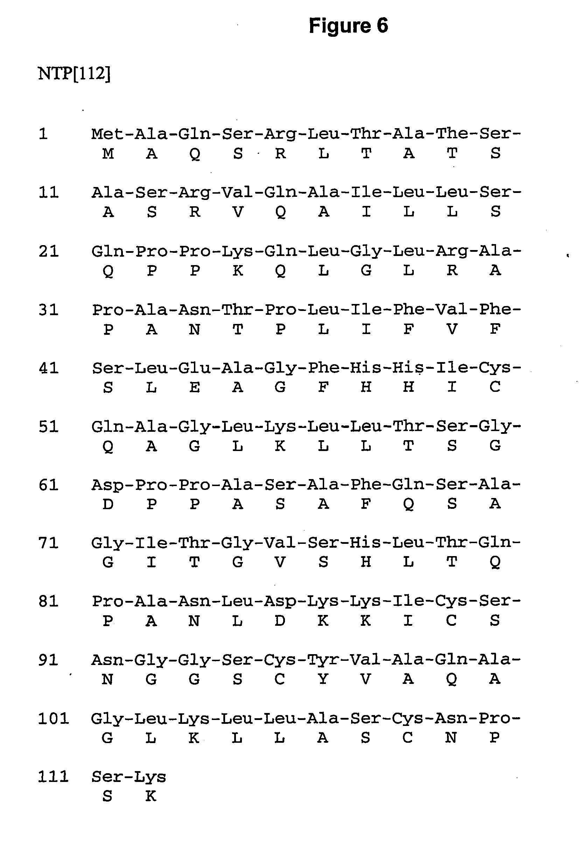

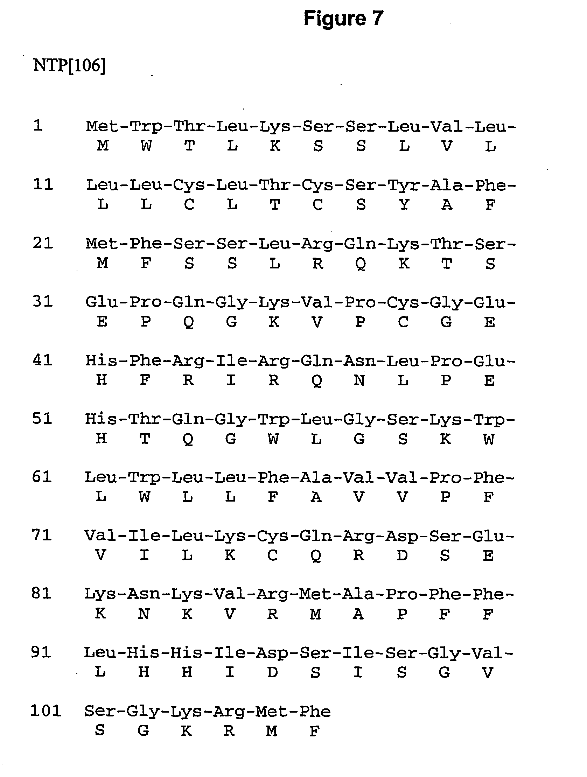

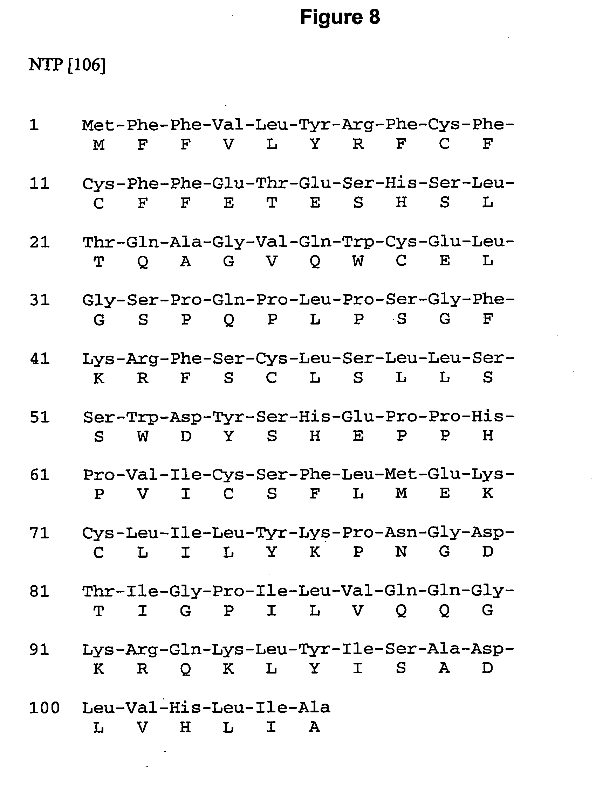

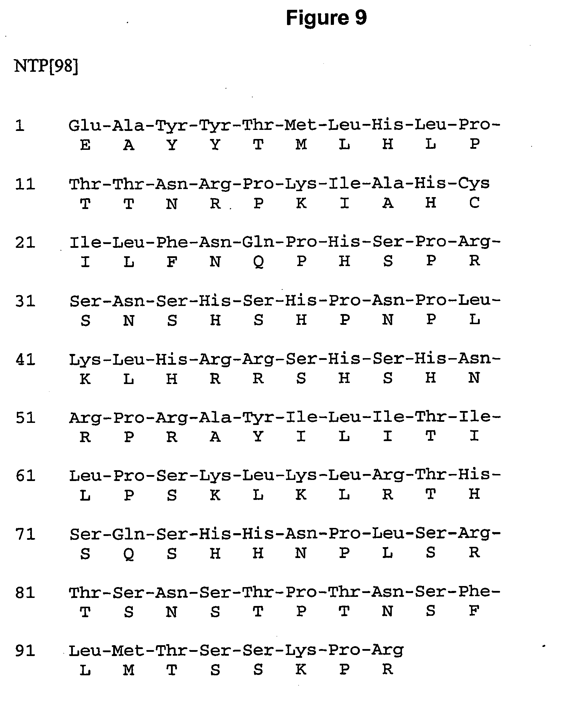

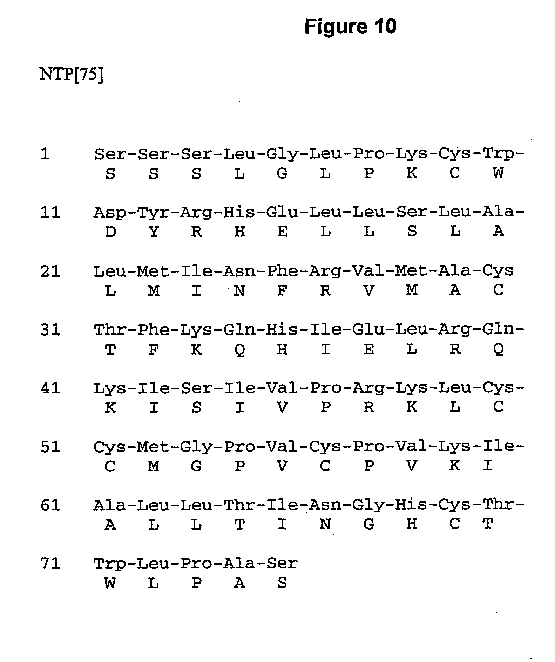

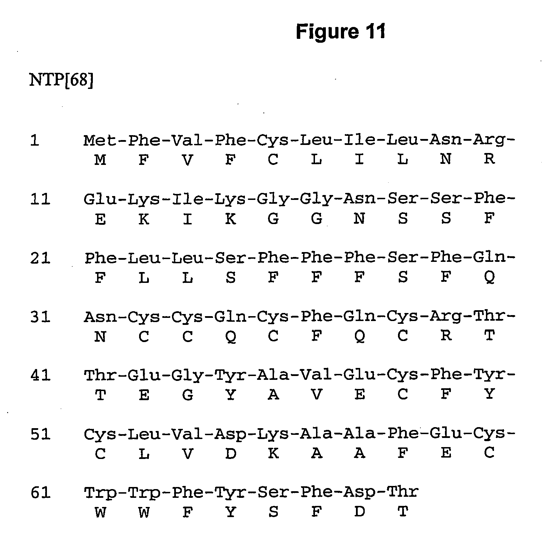

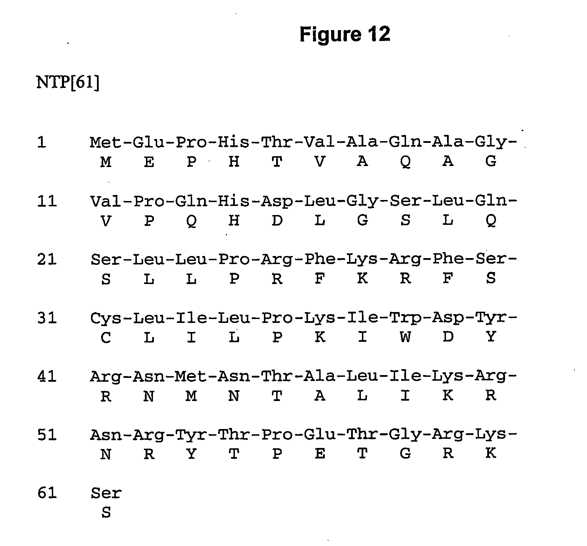

Throughout this description, the term "NTP" or "neural thread protein" refers to neural thread proteins and related molecules (including pancreatic thread protein) and the nucleic acid sequences coding for those proteins, and includes (but is not limited to) the following proteins and the nucleic acid sequences encoding the amino acid sequences for these proteins:
(a) AD7C-NTP;
(b) the ~42, ~26, ~21, ~17, ~14, and ~8 kD species of neural thread protein as described in U.S. Patent Nos. 5,948,634, 5,948,888, 5,830,670, and 6,071,705 and in de la Monte et al., J. Neuropathol. Exp. Neurol., 55(10):1038-50 (1996), de la Monte et al., J. Neurol. Sci., 138(1-2):26-35 (1996*);* de la Monte et al., J. Neurol. Sci., 135(2):118-25 (1996), de la Monte et al., J. Clin. Invest., 100:3093-3104 (1997) and de la Monte et al., Alz.. Rep., 2:327-332 (1999);
(c) proteins specifically recognized by monoclonal antibody #2 on deposit with the American Type Culture Collection, Manassas, Va., under accession number HB-12546 or monoclonal antibody #5 on deposit with the American Type Culture Collection, Manassas, Va., under accession number HB-12545;
(d) proteins coded by the AD7C-NTP gene;
(e) the 122 amino acid neural thread protein described in Sequence 40 from U.S. Patent Nos. 5,830,670, 5,948,634, and 5,948,888 and listed in NCBI Entrez-Protein Accession #AAE25447, PID g10048540, the amino acid sequences for which is illustrated in Figure 5;
(f) the 112 amino acid neural thread protein listed in NCBI Entrez-Protein Accession #XP_032307, PID g14725132, the amino acid sequences for which is illustrated in Figure 6;
(g) a 106 amino acid neural thread protein-like protein listed in NCBI Entrez-Protein Accession #AAH14951 PID g15928971, the amino acid sequences for which is illustrated in Figure 7;
(h) a 106 amino acid neural thread protein-like protein listed in NCBI Entrez-Protein Accession #XP_039102, PID g18599339, the amino acid sequences for which is illustrated in Figure 8;
(i) the 98 amino acid neural thread protein described in Sequence 30 from U.S. Patent Nos. 5,830,670, 5,948,634, and 5,948,888 and listed in NCBI Entrez-Protein Accession # AAE13612, PID g10048538, the amino acid sequences for which is illustrated in Figure 9;
(j) the 75 amino acid neural thread protein described in Sequence 48 from U.S. Patent Nos. 5,830,670, 5,948,634, and 5,948,888 and listed in NCBI Entrez-Protein Accession #AAE25448, PID g10048541, the amino acid sequences for which is illustrated in Figure 10;
(k) the 68 amino acid neural thread protein described in Sequence 36 from U.S. Patent Nos. 5,830,670, 5,948,634, and 5,948,888 and listed in NCBI Entrez-Protein Accession #AAE25446, PID g10048539, the amino acid sequences for which is illustrated in Figure 11;
(l) the 61 amino acid neural thread protein-like protein listed in NCBI Entrez-Protein Accession #AAH02534, PID g12803421, the amino acid sequences for which is illustrated in Figure 12;
(m) pancreatic thread protein;
(n) the neural pancreatic thread protein (nPTP) described in U.S. Patent No. 6,071,705; and
(o) proteins specifically recognized by the antibodies produced by a hybridoma from the group consisting of HB 9934, HB 9935, and HB 9936 deposited at the American Type Culture Collection.

The term "NTP" also includes NTP derived from mammalian tissue or produced using recombinant techniques and includes fragments, variants, derivatives, and homologs of NTP.

The term "AD7C-NTP" refers to the ~41kD protein and the gene and the nucleic acid sequences coding for it described in de la Monte et al., J. Clin. Invest., 100:3093-104 (1997), in Sequences 120 and 121 of U.S. Patent Nos. 5,948,634, 5,948,888, and 5,830,670 and in NCBI Entrez-Protein database Accession #AF010144.

As used herein, the term "antibody" refers antibodies that are capable of binding to or recognizing NTP and includes both monoclonal antibodies, which are a substantially homogeneous population, and polyclonal antibodies, which are heterogeneous populations. Polyclonal antibodies are derived from the sera of animals immunized with an antigen. Monoclonal antibodies (mAbs) to specific antigens may be obtained by methods known to those skilled in the art. *See,* for example, Kohler and Milstein, Nature 256:495-497 (1975) and U.S. Pat No. 4,376,110. Such antibodies may be of any immunoglobulin class including IgG, IgM, IgE, IgA, IgD and any subclass thereof.

The term "antibody" specifically includes (but is not limited to):
1. the monoclonal antibodies Th7, Th9, Th10, N2B10, N2I5, N2J1, N2S6, N2T8, N2U6, N3A13. N3C11, N3D12, N314, and N2-36 as previously described (Gross et al., J Clin. Invest. 76:2115-2126 (1985); Ozturk et al., Proc. Natl. Acad Sci. USA 86:419-423 (1989); de la Monte et. al., J. Clin. Invest. 86:1004-1013 (1990); de la Monte et. al., J Neurol. Sci. 113:152-164 (1992); de la Monte et al., Ann. Neurol. 32:733-742 (1992); de la Monte, S.M., et al., J Neuropathol Expl Neurol, 55:1038-1050 (1996); and de la Monte, S.M. et al, J Clin Invest 12: 3093-3104 (1997)).
2. antibodies produced by a hybridoma selected from HB 9934, HB 9935, and HB 9936, all of which are deposited at the American Type Culture Collection.
3. antibodies against neural thread proteins as described in U.S Patent No, 5,830,670, 5,948,634 and 5,948,888, including (but not limited to) mAbs Th7, Th9, Th10, Th29 and Th34 and mABs #2 and #5.
4. antibodies against a neurological form of pancreatic thread protein as described in U.S Patent No. 6,071,705, including (but not limited to) mAbs 7, 9 and 10 as described therein.

The term "antibody" as it is used herein also includes biologically active variants, homologs, peptide mimetics, reverse-D peptides, and enantiomers of an antibody to NTP.

Structurally, the simplest antibody (IgG) comprises four polypeptide chains, two heavy (H) chains and two light (L) chains inter-connected by disulphide bonds. The light chains exist in two distinct forms called kappa (κ) and lambda (λ). Each chain has a constant region (C) and a variable region (V). Each chain is organized into a series of domains. The light chains have two domains, corresponding to the C region and the other to the V region. The heavy chains have four domains, one corresponding to the V region and three domains (1, 2 and 3) in the C region. The antibody has two arms (each arm being a Fab region), each of which has a VL and a VH region associated with each other. It is this pair of V regions (VL and VH) that differ from one antibody to another (owing to amino acid sequence variations), and which together are responsible for recognizing the antigen and providing an antigen binding site (ABS). In even more detail, each V region is made up from three complementarity determining regions (CDR) separated by four framework regions (FR). The CDR's are the most variable part of the variable regions, and they perform critical antigen binding function. The CDR regions are derived from many potential germ line sequences via a complex process involving recombination, mutation and selection.

The term "antibody fragment" as it is used herein refers to a biologically active fragment of an antibody to NTP and includes (i) the Fab fragment consisting of the VL, VH, CL and CH1 domains of the antibody; (ii) the Fd fragment consisting of the VH and CH1 domains; (iii) the Fv fragment consisting of the VL and VH domains of a single arm of an antibody, (iv) the dab fragment (Ward, E. S. et al., Nature 341, 544-546 (1989) which consists of a VH domain; (v) isolated CDR regions; (vi) F(ab')₂ fragments, a bivalent fragment comprising two Fab fragments linked by a disulphide bridge at the hinge region; and (vii) single chain Fv (scFv) where the two domains of the Fv fragment, coded for by separate genes, are joined by a synthetic linker that enables them to be made as a single protein chain (known as Bird, R. E. et al., Science 242, 423-426 (1988) Huston, J. S. et al., Proc. Natl. Acad Sci., USA 85, 5879-5883 (1988)) by recombinant methods. Fab, F(ab')₂, scFv and other such fragments lack the Fc fragment of intact antibody, clear more rapidly from the circulation, and may have less non-specific tissue binding than an intact antibody (Wahl et al., J. Nucl. Med. 24:316-325 (1983)). In addition, the Fab, F(ab')₂, scFv and other such fragments may be small enough to cross the blood-brain barrier without the need for extraneous treatment of the barrier, or conjugation to barrier-penetrating conjugates, as described below.

It will be appreciated that Fab, F(ab')₂, scFv and other fragments of the antibodies useful in the present invention may be used for preventing, inhibiting, and/or ameliorating cell death and/or tissue necrosis in live tissue containing NTP in the same manner as an intact antibody may be used. Such fragments typically are produced by proteolytic cleavage, using enzymes such as papain (to produce Fab fragments) or pepsin (to produce F(ab')₂ fragments) or by recombinant methods (to produce scFv fragments).

The term "antibody fragment" as it is used herein also includes biologically active variants, homologs, peptide mimetics, reverse-D peptides, and enantiomers of antibody fragments.

The term "antibody derivative" refers to biologically active derivatives of antibodies to NTP and antibody fragments and includes derivatives of antibodies and antibody fragments produced by means of phage display technologies or bacterial or yeast cell surface display technologies. The term "antibody derivative" also includes humanized antibodies and antibody fragments and also includes biologically active variants, homologs, peptide mimetics, reverse-D peptides, and enantiomers of a biologically active antibody derivative.

The term "biologically active" refers to an antibody, antibody derivative or antibody fragment that has the ability of recognizing and/or binding to NTP and includes (but is not limited to) the ability to prevent, inhibit, reduce, control or ameliorate cell death and/or tissue necrosis caused by the presence of NTP.

An antibody is said to have "the ability of binding," be "capable of binding," "binds to" or "recognizes" a molecule (*e.g.*, a protein such as NTP) if it is capable of specifically reacting with the molecule to thereby bind the molecule to the antibody. The term "epitope" is meant to refer to that portion of any molecule capable of being bound by an antibody that can also be recognized by that antibody. Epitopic determinants usually consist of chemically active surface groupings of molecules such as amino acids or sugar side chains and have specific three dimensional structural characteristics as well as specific charge characteristics.

An "antigen" is a molecule capable of being bound by an antibody that is additionally capable of inducing an animal to produce antibody capable of binding to an epitope of that antigen. An antigen may have one, or more than one epitope. The specific reaction referred to above is meant to indicate that the antigen will react, in a highly selective manner, with its corresponding antibody and not with the multitude of other antibodies that may be evoked by other antigens.

The term "fragment" refers to a protein or polypeptide that consists of a continuous subsequence of the amino acid sequence of a NTP protein or of an antibody, antibody fragment or antibody derivative and includes naturally occurring fragments such as splice variants and fragments resulting from naturally occurring *in vivo* protease activity. Such a fragment may be truncated at the amino terminus, the carboxy terminus, and/or internally (such as by natural splicing). Such fragments may be prepared with or without an amino terminal methionine. The term "fragment" includes fragments, whether identical or different, from the same NTP protein or antibody or antibody derivative with a contiguous amino acid sequence in common or not, joined together, either directly or through a linker.

The term "variant" refers to a protein or polypeptide in which one or more amino acid substitutions, deletions, and/or insertions are present as compared to the amino acid sequence of an NTP protein or antibody, antibody derivative or antibody fragment and includes naturally occurring allelic variants or alternative splice variants of an NTP protein or antibody, antibody derivative or antibody fragment. The term "variant" includes the replacement of one or more amino acids in a peptide sequence with a similar or homologous amino acid(s) or a dissimilar amino acid(s). There are many scales on which amino acids can be ranked as similar or homologous. (Gunnar von Heijne, Sequence Analysis in Molecular Biology, p. 123-39 (Academic Press, New York, NY 1987.) Preferred variants include alanine substitutions at one or more of amino acid positions. Other preferred substitutions include conservative substitutions that have little or no effect on the overall net charge, polarity, or hydrophobicity of the protein. Conservative substitutions are set forth in Table I below.

**TABLE I**

| Conservative Amino Acid Substitutions | |
|---|---|
| Basic: | arginine |
| | lysine |
| | histidine |
| Acidic: | glutamic acid |
| | aspartic acid |
| Uncharged Polar: | glutamine |
| | asparagine |
| | serine |
| | threonine |
| | tyrosine |
| Non-Polar: | phenylalanine |
| | tryptophan |
| | cysteine |
| | glycine |
| | alanine |
| | valine |
| | proline |
| | methionine |
| | leucine |
| | isoleucine |

Table II sets out another scheme of amino acid substitution:

**TABLE II**

| Original Residue | Substitutions |
|---|---|
| Ala | gly; ser |
| Arg | lys |
| Asn | gln; his |
| Asp | glu |
| Cys | ser |
| Gln | asn |
| Glu | asp |
| Gly | ala; pro |
| His | asn; gln |
| He | leu; val |
| Leu | ile; val |
| Lys | arg; gln; glu |
| Met | leu; tyr; ile |
| Phe | met; leu; tyr |
| Ser | thr |
| Thr | ser |
| Trp | tyr |
| Tyr | trp; phe |
| Val | ile; leu |

Other variants can consist of less conservative amino acid substitutions, such as selecting residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions that in general are expected to have a more significant effect on function are those in which (a) glycine and/or proline is substituted by another amino acid or is deleted or inserted; (b) a hydrophilic residue, e.g., seryl or threonyl, is substituted for (or by) a hydrophobic residue, e.g., leucyl, isoleucyl, phenylalanyl, valyl, or alanyl; (c) a cysteine residue is substituted for (or by) any other residue; (d) a residue having an electropositive side chain, e.g., lysyl, arginyl, or histidyl, is substituted for (or by) a residue having an electronegative charge, e.g., glutamyl or aspartyl; or (e) a residue having a bulky side chain, e.g., phenylalanine, is substituted for (or by) one not having such a side chain, e.g., glycine. Other variants include those designed to either generate a novel glycosylation and/or phosphorylation site(s), or those designed to delete an existing glycosylation and/or phosphorylation site(s). Variants include at least one amino acid substitution at a glycosylation site, a proteolytic cleavage site and/or a cysteine residue. Variants also include NTP proteins or antibodies, antibody fragments or antibody derivatives with additional amino acid residues before or after the NTP or antibody amino acid sequence on linker peptides. For example, a cysteine residue may be added at both the amino and carboxy terminals of an antibody fragment in order to allow the cyclisation of the antibody fragment by the formation of a di-sulphide bond.

The term "derivative" refers to a chemically modified protein or polypeptide that have been chemically modified either by natural processes, such as processing and other post-translational modifications, but also by chemical modification techniques, as for example, by addition of one or more polyethylene glycol molecules, sugars, phosphates, and/or other such molecules, where the molecule or molecules are not naturally attached to wild-type NTP proteins or antibodies. Derivatives include salts. Such chemical modifications are well described in basic texts and in more detailed monographs, as well as in a voluminous research literature, and they are well known to those of skill in the art. It will be appreciated that the same type of modification may be present in the same or varying degree at several sites in a given protein or polypeptide. Also, a given protein or polypeptide may contain many types of modifications. Modifications can occur anywhere in a protein or polypeptide, including the peptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, crosslinking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, glycosylation, lipid attachment, sulfation, gamma-carboxylation of glutamic acid residues, hydroxylation and ADP-ribosylation, selenoylation, sulfation, transfer-RNA mediated addition of amino acids to proteins, such as arginylation, and ubiquitination. See, for instance, Proteins--Structure And Molecular Properties, 2nd Ed., T. E. Creighton, W. H. Freeman and Company, New York (1993) and Wold, F., "Posttranslational Protein Modifications: Perspectives and Prospects," pgs. 1-12 in Posttranslational Covalent Modification Of Proteins, B. C. Johnson, Ed., Academic Press, New York (1983); Seifter et al., Meth. Enzymol. 182:626-646 (1990) and Rattan et al., "Protein Synthesis: Posttranslational Modifications and Aging," Ann. N.Y. Acad. Sci. 663: 48-62 (1992). The term "derivatives" include chemical modifications resulting in the protein or polypeptide becoming branched or cyclic, with or without branching. Cyclic, branched and branched circular proteins or polypeptides may result from post-translational natural processes and may be made by entirely synthetic methods, as well.

The term "homolog" refers to a protein that is at least 75 percent identical in its amino acid sequence of an NTP protein, AD7C-NTP or an antibody, antibody derivative or antibody fragment, as the case may be, as determined by standard methods that are commonly used to compare the similarity in position of the amino acids of two polypeptides. The degree of similarity or identity between two proteins can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M Stockton Press, New York, 1991; and Carillo H. and Lipman, D., SIAM, J. Applied Math., 48: 1073 (1988). Preferred methods to determine identity are designed to give the largest match between the sequences tested. Methods to determine identity and similarity are codified in publicly available computer programs.

Preferred computer program methods useful in determining the identity and similarity between two sequences include, but are not limited to, the GCG program package (Devereux, J., et al., Nucleic Acids Research, 12(1): 387 (1984)), BLASTP, BLASTN, and FASTA, Atschul, S. F. et al., J. Molec. Biol., 215: 403-410 (1990). The *BLAST* X program is publicly available from NCBI and other sources *(BLAST* Manual, Altschul, S., *et al.,* NCBI NLM NIH Bethesda, Md. 20894; Altschul, S., et al., J. Mol. Biol., 215: 403-410 (1990). By way of example, using a computer algorithm such as GAP (Genetic Computer Group, University of Wisconsin, Madison, Wis.), the two proteins or polypeptides for which the percent sequence identity is to be determined are aligned for optimal matching of their respective amino acids (the "matched span", as determined by the algorithm). A gap opening penalty (which is calculated as 3 x (times) the average diagonal; the "average diagonal" is the average of the diagonal of the comparison matrix being used; the "diagonal" is the score or number assigned to each perfect amino acid by the particular comparison matrix) and a gap extension penalty (which is usually 1/10 times the gap opening penalty), as well as a comparison matrix such as PAM 250 or BLOSUM 62 are used in conjunction with the algorithm. A standard comparison matrix *(see* Dayhoff et al. in: Atlas of Protein Sequence and Structure, vol. 5, supp.3 [1978] for the PAM250 comparison matrix; see Henikoff et al., Proc. Natl. Acad. Sci USA, 89:10915-10919 [1992] for the BLOSUM 62 comparison matrix) also may be used by the algorithm. The percent identity then is calculated by the algorithm. Homologs will typically have one or more amino acid substitutions, deletions, and/or insertions as compared with NTP, AD7c-NTP, an antibody, antibody derivative or antibody fragment.

The term "peptide mimetic" refers to biologically active compounds that mimic the biological activity of a peptide or a protein but are no longer peptidic in chemical nature, that is, they no longer contain any peptide bonds (that is, amide bonds between amino acids). Here the term peptide mimetic is used in a broader sense to include molecules that are no longer completely peptidic in nature, such as pseudo-peptides, semi-peptides and peptoids. Examples of peptide mimetics in this broader sense (where part of a peptide is replaced by a structure lacking peptide bonds) are described below. Whether completely or partially non-peptide, peptide mimetics according to this invention provide a spatial arrangement of reactive chemical moieties that closely resemble the three-dimensional arrangement of active groups in the antibody, antibody derivative or antibody fragment on which the peptide mimetic is based. As a result of this similar active-site geometry, the peptide mimetic has effects on biological systems that are similar to the biological activity of the peptide.

The peptide mimetics of this invention are preferably substantially similar in both three-dimensional shape and biological activity to the antibodies, antibody derivatives or antibody fragments described herein Examples of methods of structurally modifying a peptide known in the art to create a peptide mimetic include the inversion of backbone chiral centers leading to D-amino acid residue structures that may, particularly at the N-terminus, lead to enhanced stability for proteolytical degradation without adversely affecting activity. An example is given in the paper "Tritriated D-ala¹ -Peptide T Binding", Smith C. S. et al., Drug Development Res., 15, pp. 371-379 (1988). A second method is altering cyclic structure for stability, such as N to C interchain imides and lactames (Ede et al. in Smith and Rivier (Eds.) "Peptides: Chemistry and Biology", Escom, Leiden (1991), pp. 268-270). An example of this is given in conformationally restricted thymopentin-like compounds, such as those disclosed in U.S. Pat. No. 4,457,489 (1985), Goldstein, G. et al. A third method is to substitute peptide bonds in the antibody, antibody fragment or antibody derivative by pseudopeptide bonds that confer resistance to proteolysis.

A number of pseudopeptide bonds have been described that in general do not affect peptide structure and biological activity. One example of this approach is to substitute retro-inverso pseudopeptide bonds ("Biologically active retroinverso analogues of thymopentin", Sisto A. et al in Rivier, J. E. and Marshall, G. R. (eds) "Peptides, Chemistry, Structure and Biology", Escom, Leiden (1990), pp. 722-773) and Dalpozzo, et al. (1993), Int. J. Peptide Protein Res., 41:561-566). According to this modification, the amino acid sequences of the peptides may be identical to the sequences of the antibody described above, except that one or more of the peptide bonds are replaced by a retro-inverso pseudopeptide bond. Preferably the most N-terminal peptide bond is substituted, since such a substitution will confer resistance to proteolysis by exopeptidases acting on the N-terminus. Further modifications also can be made by replacing chemical groups of the amino acids with Other chemical groups of similar structure. Another suitable pseudopeptide bond that is known to enhance stability to enzymatic cleavage with no or little loss of biological activity is the reduced isostere pseudopeptide bond is a (Couder, et al. (1993), Int. J. Peptide Protein Res., 41:181-184).

Thus, the amino acid sequences of these peptides may be identical to the sequences of an antibody, antibody fragment or antibody derivative, except that one or more of the peptide bonds are replaced by an isostere pseudopeptide bond. Preferably the most N-terminal peptide bond is substituted, since such a substitution would confer resistance to proteolysis by exopeptidases acting on the N-terminus, The synthesis of peptides with one or more reduced isostere pseudopeptide bonds is known in the art (Couder, *et al.* (1993), cited above). Other examples include the introduction of ketomethylene or methylsulfide bonds to replace peptide bonds.

Peptoid derivatives of NTP peptides represent another class of peptide mimetics that retain the important structural determinants for biological activity, yet eliminate the peptide bonds, thereby conferring resistance to proteolysis (Simon, et al., 1992, Proc. Natl. Acad. Sci. USA, 89:9367-9371 ). Peptoids are oligomers of N-substituted glycines. A number of N-alkyl groups have been described, each corresponding to the side chain of a natural amino acid (Simon, *et al.* (1992), cited above). Some or all of the amino acids of the antibody, antibody fragment or antibody derivative are replaced with the N-substiruted glycine corresponding to the replaced amino acid.

The term "reverse-D peptide" refers to a biologically active protein or peptide consisting of D-amino acids arranged in a reverse order as compared to the L-amino acid sequence of an antibody, antibody fragment, or antibody derivative. Thus, the carboxy terminal residue of an L-amino acid antibody protein, antibody fragment, or antibody derivative becomes the amino terminal for the D-amino acid peptide and so forth.

The term "enantiomer" refers to a biologically active protein or peptide where one or more the L-amino acid residues in the amino acid sequence of an antibody, antibody fragment, or antibody derivative is replaced with the corresponding D-amino acid residue(s).

Throughout this description, the term "amyloidosis" and the expression "cerebral amyloidosis" denotes a number of pathological conditions characterized by the deposition of abnormal fibrils ("amyloid fibrils") and/or related non-fibrillar amyloid precursor or non-precursor molecule, as well as the presence of NTP in extracellular spaces, including, for example, the Alzheimer group of diseases, namely, Alzheimer's disease (pre-senile dementia, senile dementia); Alzheimer's disease associated with Down's syndrome; familial Alzheimer's Disease; genetic Alzheimer's disease due to mutations such as Presenilin 1, Presenilin 2, and others; Alzheimer's disease associated with other central-nervous-system diseases, such as Parkinson's disease, Lewy Body Disease, and cerebrovascular diseases; congophilic angiopathy (associated or not associated with Alzheimer's disease, familial or not familial), and other disorders and diseases such as those disclosed in U.S. Patent No. 6,001,331.

Throughout this description, the expressions "amyloid plaques" and "amyloid fibrils" denote senile plaques, neuritic plaques, amyloid plaques, amyloid stars, amyloid cores, primitive plaques, classical plaques, burn out plaques, diffuse plaques, shadow plaques, neurofibrillary tangles, amyloid fibrils, paired helical filaments, and the like. Throughout this description, the term "mammal" denotes all mammals, and preferably denotes, sheep, cows, dogs, cats, apes, monkeys, mice, rats, and humans, and most preferably denotes a human.

The present invention is directed toward agents and compositions for preventing cell death and/or tissue necrosis in live tissue containing NTP. While NTP has been known and described in the literature, it was not heretofore known that NTP was a cause of cell death of cells other than the cells producing the NTP. While not intending on being bound by any theory, the present inventor believes that the presence of NTP in live tissue not only is an indication of certain nerve cell death, as previously reported, but it also is toxic insofar as it causes other live tissue cell death. The present inventor believes that NTP present in live mammalian brain tissue is a marker for Alzheimer's Disease (AD), and it exacerbates AD by causing cell death and/or tissue necrosis in the live tissue in which it exists. Accordingly, it is believed that, as a mammal becomes inflicted with AD, nerve cell death up-regulates the gene that produces NTP, thereby producing NTP at that site. The NTP so produced then begins destroying other live tissue (e.g., other nerve cells, glial cells, etc.) in the vicinity thereof, thereby exacerbating the progression of the disease. The inventor therefore believes that neutralizing the NTP with an antibody, antibody derivative or antibody fragment will help prevent, inhibit, reduce, control and/or ameliorate cell death and/or tissue necrosis in live tissue that contains NTP or that has been contacted by NTP.

The ability of NTP to cause cell death and/or tissue necrosis in live tissue where it is present makes it useful for removing or destroying harmful or unwanted tissue or cellular elements such as benign or malignant tumors in humans. The inventor anticipates that it would be useful to modulate, control, prevent or inhibit the cell death and/or tissue necrosis caused by the administration of NTP for such a purpose, particularly to avoid or reduce cell death or tissue necrosis in surrounding tissue. The inventor therefore believes that neutralizing the NTP with an antibody, antibody derivative or antibody fragment will help modulate, control, prevent or inhibit cell death and/or tissue necrosis either in or near live tissue where NTP has been administered.

Methods of making NTP recombinantly or otherwise are disclosed in, for example, U.S. Patent Nos. 5,948,634, 5,948,888, 5,830,670, and 6,071,705. Raising antibodies against NTP so that one can diagnose AD and other associated disorders and diseases, also is disclosed in these documents. It will be evident to those skilled in the art that one may use NTP fragments, homologs, derivatives and variants, as well as NTP from diverse sources (*e.g.*, natural, pancreatic, purified, synthesized, or from different expression systems *in vitro,* etc.) to make or screen for any of the antibodies, antibody fragments or antibody derivatives useful in the present invention.

The use of NTP fragments to raise or screen for antibodies, antibody derivatives or antibody fragments also is encompassed by the scope of the invention. Depending on the purpose, it is common to utilize a smaller active fragment of a larger active molecule. The use of molecules of the NTP family, such as AD7C-NTP and other neural thread proteins and pancreatic thread proteins, is also encompassed by the scope of the invention.

The monoclonal antibodies, particularly mAbs Th7, Th9, Th10, and N314, useful in the present invention, may be prepared as previously described (Gross et al., J. Clin. Invest. 76:2115-2126 (1985); Ozturk et al., Proc. Natl. Acad. Sci. USA 86:419-423 (1989); de la Monte et. al., J Clin. Invest. 86:1004-1013 (1990); de la Monte et. al., J. Neurol Sci. 113:152-164 (1992); de la Monte et al., Ann. Neurol. 32:733-742 (1992); and de la Monte, S.M., et al., Journal of Neuropathology and Experimental Neurology, 55:1038-1050 (1996)). The Th monoclonal antibodies were generated against the purified pancreatic form of thread protein (*Id.*). NTP-specific polyclonal and monoclonal antibodies can also be generated against a substantially pure NTP isolated from recombinant hosts (for example, *see* Carroll et al., "Production and Purification of Polyclonal Antibodies to the Foreign Segment of β-Galactosidase Fusion Proteins," in DNA Cloning: A Practical Approach, Volume III, IRL Press, Washington, D.C., pp. 89-111 (1987); Mole et al., "Production of Monoclonal Antibodies Against Fusion Proteins Produced in Escherichia coli," in DNA Cloning: A Practical Approach, Volume III, IRL Press, Washington, D.C., pp. 113-1139 (1987)). Alternatively, NTP-specific polyclonal and monoclonal antibodies can be generated against a substantially pure NTP isolated from biological material such as brain tissue and cell lines, by using well known techniques.

For example, monoclonal antibodies specific for the various NTP molecules of approximately, 8, 14, 17, 21, 26, and 42 kDa molecular weights may be prepared from recombinant-derived proteins, which are expressed, isolated, and purified from the cDNA (*i.e.,* 1-9a), genomic clones (G2-2 PstI) and AD-NTP 3-4 cDNA clones. These NTP molecules can be derived from the above cDNAs and genomic clones, inserted and produced in suitable expression vectors. Since there are regions of 60-70% homology in the 5' ends of the 1-9a NTP cDNA and PTP, one can obtain monoclonal antibodies that bind specifically to the NTP recombinant proteins and not to the pancreatic form by performing routine differential screening (*see,* for example, de la Monte et al., J. Clin. Invest. 86: 1004-1013 (1990)). Although there will be monoclonal antibodies that bind to both NTP and PTP, it will be possible to generate NTP-specific monoclonal antibodies because there is a substantial sequence divergence between NTP molecules of various forms (e.g., 8, 14, 17, 21, 26, and 42 kDa) and because an epitope may be defined by as few as 6-8 amino acids. In the present invention, monoclonal antibodies that bind to both NTP and PTP can be used in the present invention so long as they are capable of inhibiting, preventing, and/or ameliorating cell death and/or tissue necrosis in live tissue that contains NTP.

The antibodies may also be prepared by using chimeric or transgenic animals such as mice developed by Abgenix using its Xenomouse technology (U.S. Patent Nos. 6,162,963, 6,150,584, 6,114,598, 6,075,181, and 5,939,598) or HuMAb-Mouse, Kirn TC Mouse or KM-Mouse mice developed by Medarex (U.S. Patent Nos. 6,300,129, 5,877,397, 5,874,299, 5,814,318, 5,789,650, 5,770,429, 5,661,016, 5,633,425, 5,625,126, 5,569,825, and 5,545,806).

Antibody fragments may be prepared by those ordinarily skilled in the art using well-known techniques. Antibody derivatives may be prepared from biologically active antibodies, antibody fragments or other antibody derivatives using phage display, bacterial cell surface display or yeast cell surface display technologies in order to create combinatorial peptide libraries. NTP can then be used to screen such libraries to identify biologically active antibodies, antibody fragments or antibody derivatives.

The use of filamentous phage display vectors, referred to as phagemids, is a well-known method for the efficient preparation of large libraries of monoclonal antibodies having diverse and novel imnunospecificites, The technology uses a filamentous phage coat protein membrane anchor domain as a means for linking gene-product and gene during the assembly of filamentous phage replication, and has been used for the cloning and expression of antibodies from combinatorial libraries (Kang, et al, Proc. Natl. Acad. Sci., U.S.A., 88:4363, 1991, Barbas, et al., Proc. Natl. Acad Sci., U.S.A., 88:7978, 1991).

In phage display, the protein of interest (an antibody, antibody fragment or antibody derivative in this case) is expressed as a polypeptide fusion to a bacteriophage coat protein and subsequently screened by binding to immobilized or soluble biotinylated ligand (in this case NTP) in a process called "panning." Phage bearing nonspecific antibodies can be removed by washing, and then the bound phage are eluted and amplified by infection of *E. coli.* Phage display has been successfully applied to generate antibodies against many antigens, including hepatitis B surface antigen; polysaccharides, insulin-like growth factor 1, 2-phenyloxazol-5-one, and 4-hydroxy-5-iodo-3-nitro-phenacetyl-(NIP)-caproic acid.

Yeast and bacterial cell surface display technologies are other methods in which a diverse array of proteins, including antibodies and antibody fragments, may be displayed and then screened for those that exhibit favorable characteristics, such as the ability to bind to an antigen with the desired affinity and specificity. (Bader, ET et al, Nat Biotechnol 15: 553-557 (1997); U.S.Patent No. 6,300,065 ; Francisco, JA et al, Proc Natl Acad Sci USA 89: 2713-2717 (1992); Georgiou G, et al, Nat Biotech 15: 29-34 (1997)).

It may be necessary to humanize monoclonal antibodies produced using mammals that are not humans. The use of such non-human antibodies in vivo in humans can lead to problems. The foreign immunoglobulins can elicit an anti-globulin response (known as a human anti-mouse antibody (HAMA) response) that can interfere with therapy (R. A. Miller et al, Blood 62 988-995 (1983)) or cause allergic or immune complex hypersensitivity (B. Ratner, Allergy, Anaphylaxis and Immunotherapy Williams and Wilkins, Baltimore (1943)).

One approach to overcoming these problems is to humanize such antibodies by modifying them accordingly. Two general methods of humanization have been developed: (i) loop-grafting, where the CDR loops are grafted directly onto the human Fv framework found to most closely resemble the sequence of the non-human animal; and (ii) resurfacing, where those Fv framework residues that are most exposed on the surface are mutated to their human counterpart. Winter and colleagues (GB2188638B) developed one such method. The complementarity determining regions (CDRs) of the mouse antibody, which comprise the antigen combining site, are inserted into human framework regions thereby generating antibodies in which only the CDR sequences are derived from the original mouse antibody. Herceptin and Rituxan are two successful examples of humanized antibodies that have been approved for therapeutic use. Combinatorial methods employing such technologies as phage display can also be used to generate humanized antibodies and antibody fragments as disclosed, for example, in U.S. Patent No. 5,565,332.

The use of cloned conjugates of antibody fragments targeted to recombinant NTP or cloned conjugates of antibody-like proteins targeted to recombinant NTP also is encompassed by the scope of the invention. The advantages of a cloned conjugate targeted to NTP or a related molecule include manufacturing and standardized production of the cloned conjugated molecule.

Methods of making and detecting such antibodies, antibody fragments, or antibody derivatives are well known to those of ordinary skill in the art, and are described in more detail below. Standard reference works setting forth the general principles of immunology include the work of Klein (Immunology: The Science of Self-Nonself Discrimination, John Wiley & Sons, New York (1982)); Kennett et al. (Monoclonal Antibodies and Hybridomas: A New Dimension in Biological Analyses, Plenum Press, New York (1980)); Campbell ("Monoclonal Antibody Technology," In: Laboratory Techniques in Biochemistry and Molecular Biology, Volume 13 (Burdon, R., et al., eds.), Elsevier, Amsterdam (1984)); and Eisen (In: Microbiology, 3rd Ed. (Davis, et al., Harper & Row, Philadelphia (1980)).

The antibodies, antibody derivatives or antibody fragments useful in the present invention also can be conjugated to various labeling components, or radioactive components. In this embodiment of the invention, the labeled antibody can be administered, bind to NTP in live tissue, detected using various techniques known in the art, and then various types of radiation can be employed in a precise, controlled manner to neutralize or kill the NTP, thereby achieving the same effect. Various methods of labeling NTP-antibodies and fragments, as well as their detection are disclosed in U.S. Patent Nos. 5,948,634, 5,948,888, 5,830,670, and 6,071,705. Radioactive-labeled antibodies, antibody derivatives and antibody fragments also can be used as described above, with the exception that the radioactive portion of the antibody conjugate may serve to neutralize or otherwise kill the NTP without the need for exogenous radiation.

The antibodies, antibody derivatives or antibody fragments preferably are contacted with live tissue containing NTP. Any live tissue that contains NTP, or that might at some point in time contain NTP is encompassed by the present invention. Preferably, the tissue is tissue selected from mammalian tissue.

An embodiment of the invention includes use of at least one antibody, antibody fragment or antibody derivative that recognises NTP for the manufacture of an agent for treating conditions caused by necrosis of live tissue due to the presence ofNTP. In this context, the necrosis of live tissue and cell death due to the presence of NTP denotes cell death and/or tissue necrosis of cells other than the dying cells that produce the NTP. In the use an antibody, antibody derivative or antibody fragment as described above that binds to or otherwise recognizes NTP is used to manufacture an agent to be administered to a mammal suffering from such a condition in an amount and for a period of time sufficient to prevent and/or inhibit the live tissue necrosis caused by the presence of NTP.

The treatment of nervous system disorders or other brain-related disorders can be achieved by administering drugs that affect nervous system function or dysfunction in animals or patients. Typically, such drugs are administered by peripheral application, either via the oral or the systemic route. While some drugs are able to cross the blood brain barrier (bbb), others do not pass the bbb efficiently or not at all and are only effective when given directly into the brain. The term "blood-brain barrier" or "bbb", as used herein, refers to the bbb proper as well as to the blood-spinal barrier. The blood-brain barrier, which consists of the endothelium of the brain vessels, the basal membrane and neuroglial cells, acts to limit penetration of substances into the brain. Sometimes the structure of the bbb is subdivided into two components: the endothelial or capillary barrier and the ependymal barrier. Banks, W. A., Kastin, A. J., Barrera, "Delivering peptides to the central nervous system: Dilemmas and strategies," Pharm. Res. 8:1345-1350 (1991).

The nature of the substance penetration through the bbb has not yet been determined but it is known that many of the regulators of brain function such as cytokines, transferrin, encephalins and endorphines can pass through the bbb from the blood vessels into the brain. Raeissi, S., Audus, J., "In vitro characterization of blood-brain barrier permeability to delta sleep-inducing peptide." J. Pharm. Phy. 41:848-852(1989); Zlokovich, B., Susie, V. T., Davson, H. Begley, D. J., Jankov, R. M., Mitrivic, B. M., Lipovac, M. N., "Saturable mechanism for delta sleep-inducing peptide (DSIP) at the blood-brain barrier of the vascularly perfused guinea pig brain." Peptides 10:249-254(1989); and Zlokovich, B., "In vivo approaches for studying peptide interaction at the blood-brain barrier." J. Control. Rel. 13:185-201(1990). However, many substances that can affect the Central Nervous System (or CNS) such as adenosine, β-endorphin, synthetic analogs of endogenous peptides Houghten, R. A. Swann, R. W., Li, C. H., "β-Endorphin: Stability, clearance behavior and entry into the central nervous system after intravenous injection of the tritiated peptide in rats and rabbits." Proc. Natl. Acad Sci. USA 77:4588-4591(1980); Levin, E. R., Frank, H. J. K., Weber, M. A., Ismail, M., Mills M., "Studies on penetration of the blood-brain barrier by atrial natriuretic factor." Biochem. Biophys. Res. Commun. 147:1226-1231(1987) Sakane, T., Tanaka, C., Yamamoto, A., Hashida, M., Sesaki, H., Ueda, H., Takagi, H., "The effect of polysorbate 80 on brain uptake and analgesic effect of D-kyoto." Int. J. Pharm. 57:77-83(1989), as well as some excitatory and inhibitor amino acids and trophic factors, penetrate poorly or not at all through the bbb. At present, drugs with no bbb penetration or poor bbb penetration can only be given by direct CNS infusion or by implantation of controlled-release polymers. *(See,* e.g., U.S. Pat. No. 4,883,666, Sabel et al.*).*

One way to overcome some of the limitations of traditional drug therapy is to increase the relative amount of drug that passes the bbb. The belief is that if one can increase the amount of the drug crossing the bbb while reducing the peripheral dose of a given drug or diagnostic substance, the peripheral side effects of the drug are also less severe, while at the same time maintaining the desired effect in the brain. A number of approaches have been described as effective in increasing drug penetration through the bbb.

One approach has been to alter the function of the bbb itself. For instance, osmotic agents, when given peripherally (such as by intravenous injection), result in the opening of the bbb. Further, some drugs acting on the CNS can change the permeability of the bbb for other substances; cholinomimetic arecolines, for instance, have been reported to induce changes of drug penetration through the bbb. Saija, A., Princi, P., De Pasquale, R., Costa, G., "Arecoline but not haloperidol produces changes in the permeability of the blood-brain barrier in the rat." J. Pharm. Pha. 42:135-138 (1990).

Other drugs that can be administered to alter the permeability of the bbb are disclosed in U.S. Pat. Nos. 5,059,415 and 5,124,146, both issued to E. A. Neuwelt. Bradykinin is one specific drug with such effects. (U.S. Pat. No. 5,112,596, issued to Malfroy-Camine). Another method comprises administering permeabilizer peptides such as A-7 or conformational analogs thereof. (WO 92/18529, an application of J. W. Kozarich et al.). A relatively invasive method has been proposed by A. Tomasz and E. Tuomanen (WO 91/16064) who administer parenteral injections of purified cell wall or cell wall fragments of eubacteria such as *Streptococcus pneumoniae* to open the bbb.

U.S. Pat. No. 5,260,210 issued to L. L. Rubin et al.*,* discloses a method whereby the permeability of the blood-brain barrier is increased by administering an agent that reduces or interferes with cyclic AMP concentrations or that increases cyclic GMP concentrations.

Another approach is the modification of the drug molecules themselves. For instance, macromolecules, such as proteins, may not pass the bbb at all, or may pass through with difficulty or with alterations that adversely impact the proteins efficacy. For example, one can first isolate the macromolecule active site, i.e., the portion of the molecule that triggers the biologically desirable event, and then use only this active site. Since size is one of the factors in allowing permeability of the bbb, the reduced size can be used so that the smaller molecule can now pass the bbb. Use of antibody fragments therefore is desirable in this context. Other modifications to macromolecules to attempt passage of the bbb include glycating the proteins, thereby enhancing their permeability of the bbb, or forming a prodrug. U.S. Pat. No. 5,260,308, issued to J. F. Podusio and G. L. Curran, discusses glycating proteins, while U.S. Pat. No. 4,933,324 and WO 89/07938, both on applications of V. E. Shashoua, disclose formation of a prodrug. These prodrugs are formed from a fatty acid carrier and a neuroactive drug which is unable to pass across the bbb on its own. A similar system is disclosed in WO 89/07938.

Still another approach is the implantation of controlled release polymers that release the active ingredient from a matrix-system directly into the nervous tissue. However, this approach is invasive and requires surgical intervention if implanted directly into the brain or spinal cord (see Sabel et al. U.S. Pat. No. 4,883,666; and Sabel et al. U.S. patent application Ser. No. 07/407,930). It also is known to administer compositions directly to internal portions of the brain, as disclosed on U.S. Pat. No. 5,800,390.

These methods enable the delivery of sustained release, solid preparations and semi-solid preparations directly to brain tissue.

To overcome these limitations, another approach has been tried in which drug carrier systems are used such as liposomes, erythrocyte ghosts, antibody-conjugates, and monoclonal antibody conjugates. One of the major problems in targeted drug delivery is the rapid opsonization and uptake of injected carriers by the reticuloendothelial system (RES), especially by the macrophages in the liver and spleen. This obstacle may be partially overcome in the case of liposomes by incorporation of so-called "stealth" lipids, such as phosphatidylinositol, monosialoganglioside, or sulfogalactosylceramide.

U.S. Pat. Nos. 5,182,107 and 5,154,924, both issued to P. M. Friden, teach a method of conjugating a drug with an antibody where the antibody is reactive with a transferrin receptor. Transferrin receptors are located on brain capillary endothelial cells, which thus can transport a drug, such as nerve growth factor, across the bbb. U.S. Pat. No. 5,004,697 (issued to Pardridge) improves such an antibody-conjugate method by providing cationized antibodies with a specific isoelectric point (see also WO 89/01343 by Pardridge).

Another approach is to create chimeric peptides to which the active agents are conjugated (U.S. Pat. No. 4,801,575, also issued to Pardridge). Such a system is further discussed also in U.S. Pat. No. 4,902,505, issued to Pardridge and Schimmel, in which the chimeric peptide, such as histone, is capable of crossing the bbb by transcytosis.

U.S. Pat. Nos. 5,187,158 and 5,017,566, both issued to N. S. Bodor, disclose a brain-specific drug delivery method wherein a centrally acting drug is given with the reduced, biooxidizable lipoidal form of a dihydropyridine reaction-pyridine salt redox carrier such as dopamine. (*See also* U.S. Pat. No. 4,880,816, also issued to Bodor).

A rather invasive approach is taken to deliver genetic material to the brain. This is done, for example, by chemically disrupting the bbb and then using viruses to deliver genes across the bbb. *(See,* U.S. Pat. No. 4,866,042, issued to E. A. Neuwelt). Here, a corrective genetic material is incorporated into a virus and the virus is then injected into the bloodstream.

Finally, yet another carrier system to deliver drugs across the bbb is the use of liposomes, as disclosed by F. D. Collins and R C. Thompson (WO 91/04014). Here, liposomes are targeted to specific endogenous brain transport systems that transport specific ligands across the bbb.

Another approach is disclosed in U.S. Patent No. 6,117,454, to Kreuter, et al. The subject matter of the Kreuter patent includes a method, composition and drug targeting system using surfactant coated nanoparticles as a drug carrier (or targeting molecule) for a wide range of drugs in order to enhance the penetration of drugs or diagnostic agents across the bbb.

The art therefore proposes a number of different approaches to detecting AD and the presence of NTP in the brain, as well as a number of approaches to rendering drugs or other therapeutics accessible to the brain by allowing them to traverse the bbb. The art does not recognize the importance of preventing cell death and/or tissue necrosis caused by NTP in AD brain or in other tissue.

Those skilled in the art will appreciate that instead of directly administering the antibody, the antibody can be produced or expressed by the mammal through gene expression (e.g., gene therapy) or through a vaccine. Skilled artisans are capable of creating, isolating and purifying suitable genes or vaccines useful in inducing antibody or antibody fragment expression, using the guidelines provided herein.

For example, gene therapy has attracted wide attention as a method to treat various mammalian diseases and enhance production of specific proteins or other cellular products. Gene therapy generally is accomplished by introducing exogenous genetic material into a mammalian patient's cells. The introduced genetic material can be designed to replace an abnormal (defective) gene of the mammalian patient ("gene replacement therapy"), or can be designed for expression of the encoded protein or other therapeutic product without replacement of any defective gene ("gene augmentation"). Because many congenital and acquired medical disorders result from inadequate production of various gene products, gene therapy provides a means to treat these diseases through either transient or stable expression of exogenous nucleic acid encoding the therapeutic product.

Gene therapy can be accomplished by either direct transformation of target cells within the mammalian subject *(in vivo* gene therapy) or transformation of cells *in vitro* and subsequent implantation of the transformed cells into the mammalian subject (*ex vivo* gene therapy). *In vivo* gene therapy is particularly preferred for use in the present invention. In addition to repair of somatic cells, it is generally known that *in vivo* gene therapy also can be used for systemic treatment, an area in which gene therapy has broad applications. Systemic treatment involves transfecting target cells with the DNA of interest, expressing the coded protein in that cell, and the capability of the transformed cell to subsequently secrete the manufactured protein into blood.

A variety of methods have been developed to accomplish *in vivo* transformation including mechanical means (e.g, direct injection of nucleic acid into target cells or particle bombardment), recombinant viruses, liposomes, and receptor-mediated endocytosis (RME) (for reviews, see Chang et al. 1994 Gastroenterol. 106:1076-84; Morsy et al. 1993 JAMA 270:2338-45; and Ledley 1992 J. Pediatr. Gastroenterol. Nutr. 14:328-37).

Suitable methods of developing and administering genes and vaccines suitable to induce *in vivo* expression of the antibody or antibody fragments that recognize or bind to NTP are disclosed in, for example, U.S. Patent Nos. 6,210,919 and 6,225,290.

Any condition caused by cell death and necrosis of live tissue due to the presence of NTP can be treated in accordance with the present invention. Preferably, the condition is selected from AD, stroke, brain tumor, and other brain diseases, in particular, neurodegenerative diseases (such as AD, Pick's Disease, Lewy body Disease, Parkinson's Disease, etc.).

In accordance with preferred embodiments of the invention, at least one antibody, antibody derivative or antibody fragment that binds to NTP in an amount sufficient to prevent cell death and/or tissue necrosis caused by the presence of the NTP is used to manufacture an agent which can be used to contact live tissue containing NTP. Methods of administering an antibody, antibody derivative or antibody fragment that binds to NTP include administering the antibody intramuscularly, orally, intravenously, intrathecally, intranasally, topically, transdermally, etc. In addition, the antibody, antibody derivative or antibody fragment can be expressed or produced *in vivo* by administration of a gene that expresses the protein, or by administration of a vaccine that induces such production, as described above. Further, the antibody, antibody derivative or antibody fragment can be expressed or produced *in vivo* by administration or introduction of a cell, bacteria or virus that expresses the protein *in vivo*.

Solid dosage forms for oral administration include capsules, tablets, pills, powders, and granules. In such solid dosage forms, the active compound usually is admixed with at least one of the following: (a) one or more inert excipients (or carrier), such as sodium citrate or dicalcium phosphate; (b) fillers or extenders, such as starches, lactose, sucrose, glucose, mannitol, and silicic acid; (c) binders, such as carboxymethylcellulose, alginates, gelatin, polyvinylpyrrolidone, sucrose and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as acetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the active antibody compounds, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like. Besides such inert diluents, the composition can also include adjuvants, such as wetting agents, emulsifying and suspending agents, sweetening, .flavoring, and perfuming agents.

Another method of administering the antibody that recognizes or binds to NTP is by a transdermal or transcutaneous route. One example of such an embodiment is the use of a patch. In particular, a patch can be prepared with a fine suspension of antibody in, for example, dimethylsulfoxide (DMSO), or a mixture of DMSO with cottonseed oil and brought into contact with the skin of the mammals away from the location site where the tissue containing NTP exists. The composition may be present inside a skin pouch. Other mediums or mixtures thereof with other solvents and solid supports would work equally as well. The patch can contain the antibody or antibody fragment in the form of a solution or a suspension. The patch can then be applied to the skin of the patient, for example, by means of inserting it into a skin pouch of the patient formed by folding and holding the skin together by means of stitches, clips or other holding devices. This pouch should be employed in such a manner so that continuous contact with the skin is assured without the interference of the mammal. Besides using a skin pouch, any device can be used which ensures the firm placement of the patch in contact with the skin. For instance, an adhesive bandage could be used to hold the patch in place on the skin.

Actual dosage levels of the active ingredients in the compositions of the invention may be varied to obtain an antibody-containing composition that is effective to obtain a desired tissue necrosis-inhibiting therapeutic response for a particular composition and method of administration. The selected dosage level therefore depends upon the desired therapeutic effect, the route of administration, the desired duration of treatment, and other factors.

With mammals, including humans, the effective amounts can be administered on the basis of body surface area. The interrelationship of dosages for animals of various sizes, species, and humans (based on mg/M² of body surface) is described by E. J. Freireich et al., Cancer Chemother. Rep., 50(4):219 (1966). Body surface area may be approximately determined from the height and weight of an individual (*see e.g.,* Scientific Tables, Geigy Pharmaceuticals, Ardsley, N.Y. pp. 537-538 (1970)).

The total daily dose of the antibody, antibody derivative or antibody fragment that binds to NTP that is administered to a host may be in single or divided doses. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, potency of the administered drug, rates of absorption and excretion, combination with other drugs and the severity of the particular disease being treated.

The antibody-containing compositions of the present invention preferably contain a component that enables the antibody to cross the blood-brain barrier to treat live brain tissue containing NTP. Any of the variety of components described above can be used to render the antibodies capable of crossing the blood-brain barrier. For example, only a small binding portion or fragment of the antibody (e.g., the F(ab) or F(ab')₂ portion) may be used, whereby the fragment is small enough to traverse the blood-brain barrier. In this case, no additional component would be required.

The antibody, antibody derivative or antibody fragment may be glycated to enhance the permeability of the bbb, as disclosed in U.S. Pat. No. 5,260,308, or formed into a prodrug, as disclosed in U.S. Pat. No. 4,933,324 and WO 89/07938. These prodrugs preferably are formed from a fatty acid carrier and an antibody that binds or otherwise recognizes NTP, which is unable to pass across the bbb on its own.

An alternative approach is the implantation of controlled release polymers that release the antibody, antibody derivative or antibody fragment from a matrix-system directly into the nervous tissue (see Sabel et al. U.S. Pat. No. 4,883,666; and Sabel et al. U.S. patent application Ser. No. 07/407,930.). It also is possible to use drug carrier systems such as liposomes, erythrocyte ghosts, antibody-conjugates, and monoclonal antibody conjugates. In accordance with this , so-called "stealth" lipids, such as phosphatidylinositol, monosialoganglioside, or sulfogalactosylceramide can be used to form the liposomes containing the aforementioned antibodies and antibody conjugates.

The antibodies, antibody derivatives or antibody fragments that bind to NTP may be conjugated with another antibody that is reactive with a transferrin receptor, as disclosed in U.S. Pat. Nos. 5,182,107 and 5,154,924. Transferrin receptors are located on brain capillary endothelial cells, which thus transport a drug, such as nerve growth factor, or other antibodies such as those raised against NTP, across the bbb. The antibody-antibody conjugate described above can be further enhanced by providing cationized antibodies with a specific isoelectric point, as disclosed in U.S. Pat. No. 5,004,697 and WO 89/01343.

Another embodiment of the invention encompasses creating chimeric peptides to which the active antibody, antibody derivative and/or antibody fragment is conjugated, as disclosed in U.S. Pat. No. 4,801,575. The chimeric peptide preferably is histone, which is capable of crossing the bbb by transcytosis, as disclosed in U.S. Pat. No. 4,902,505. A further embodiment of the invention includes providing the antibody or antibody fragment together with the reduced, biooxidizable lipoidal form of a dihydropyridine reaction-pyridine salt redox carrier such as dopamine, as disclosed in U.S. Pat. Nos. 4,880,816, 5,187,158, and 5,071,566.

Another approach also can be taken to deliver the antibody, antibody derivatives or antibody fragments to the brain. This can be done by chemically disrupting the bbb and then using viruses to deliver the antibody, antibody derivative(s) or antibody fragment(s) across the bbb, as disclosed in U.S. Pat. No. 4,866,042. Here, it is preferred that a corrective genetic material is incorporated into a virus and the virus is then injected into the bloodstream. Yet another carrier system that can be used to deliver the antibody, antibody derivative(s) or antibody fragment(s) across the bbb is the use of liposomes, as disclosed by F. D. Collins and R. C. Thompson (WO 91/04014). Here, liposomes preferably are targeted to specific endogenous brain transport systems that transport specific ligands across the bbb. Surfactant coated nanoparticles also can be used as a drug carrier (or targeting molecule) for the antibody, antibody derivative(s) or antibody fragment(s) of the invention in order to enhance the penetration thereof across the bbb, as disclosed in U.S. Patent No. 6,117,454.

Another approach is to use L-amino acid oxidase to reduce the plasma level of the antibody, antibody derivative or antibody fragment to allow transport of the antibody, derivative or fragment across the bbb. Such an approach is described in more detail in U.S. Patent No. 5,695,751.

Another approach in accordance with the uses of the present invention is to administer compositions comprising the antibody, derivative or fragment locally. Devices useful in administering compositions to an internal portion of the brain are described in, for example, U.S. Patent No. 5,800,390. Sustained release, solid preparations and semi-solid preparations can be administered directly to brain tissue. Such administration can be accomplished by inserting a needle-like member of such an intracerebral device that is optionally implanted in the head so that a distal end of the guide is positioned at a site of administration.

A preferred composition of the present invention for administration to a mammal suffering from a condition caused by cell death and/or tissue necrosis duc to the presence of NTP contains the antibody, antibody derivative and/or antibody fragment that recognizes NTP, and a component that enables the antibody, derivative or fragment to cross the bbb. Other compositions include a gene that expresses the antibody, antibody derivative and/or antibody fragment, and a component that enables the gene to cross the bbb. An additional composition includes a vaccine that induces expression of the antibody, antibody derivative and/or antibody fragment, and a component that enables the vaccine to cross the bbb.

It is preferred in the present invention that the amount of antibody, antibody derivative or antibody fragment that contacts the live tissue containing NTP be an amount sufficient to inhibit, prevent, and/or ameliorate cell death and/or tissue necrosis caused by NTP. The specific amount can be determined by those skilled in the art, using the guidelines provided herein. It is preferred that enough antibody, antibody derivative or antibody fragment be administered to reduce cell death or tissue necrosis by more than 50%, when compared to a control where no antibody is present and cell death caused by NTP goes unchecked. More preferably, the antibody, antibody derivative or antibody fragment is administered to reduce cell death or tissue necrosis by more than 60%, even more preferably by more than 70%, and most preferably by more than 75%, when compared to a control where no antibody is present and cell death or tissue necrosis caused by NTP goes unchecked.

Such an amount will invariably depend on the particular type of tissue, the antibody, antibody derivative or antibody fragment used, as well as the amount of NTP. Using the methods disclosed in any of the aforementioned U.S. Patent Nos. 5,948,634, 5,948,888, 5,830,670, and 6,071,705, one can estimate the relative amount of NTP present, and then conduct a series of *in vitro* experiments using mammalian tissue obtained from various sources, such as any of those disclosed in the aforementioned U.S. patents, determining the amount ofNTP in the tissue, and then determining the requisite amount of antibody, antibody derivative or antibody fragment that binds to NTP chat is required to obtain the requisite degree of cell death or tissue necrosis prevention (*i.e.*, preferably by more than 60% when compared to a control). Skilled artisans are capable of carrying out these experiments without undue experimentation, using techniques known in the art, as well as using the guidelines provided herein.

The amount of antibody, antibody derivative or antibody fragment to be administered to the mammal that has the tissue containing NTP then can readily be determined based on the body weight of the mammal, and the expected delivery amount to the tissue. The amount of antibody, antibody derivative or antibody fragment that will be expected to be delivered to the brain tissue of a mammal will depend on the particular mechanism that is employed to render the antibody, derivative or fragment capable of crossing the bbb. The same holds true for administration of genes that express the antibody, derivative or fragment, or for the administration of vaccines that induce expression or production of the antibody, derivative or fragment. Again, those skilled in the art are capable of determining without undue experimentation the appropriate dose of gene, vaccine, antibody, antibody derivative or antibody fragment to administer to a mammal using the techniques described in the aforementioned patents and by using the guidelines provided herein.

The following examples are provided to illustrate the present invention. It should be understood, however, that the invention is not to be limited to the specific conditions or details described in these examples.

### Example 1

This example demonstrates cell death in live tissue *(in vivo*) due to the presence of AD7C-NTP.

AD7C-NTP was obtained in accordance with the procedures outlined in any one of U.S. Patent Nos. 5,948,634, 5,948,888, 5,830,670, and 6,017,705.

Eight normal rats were injected in the skin and subcutaneously, each in 3 different foci, with purified recombinant AD7C-NTP in saline at concentrations of 0.1-1.0 µg/mL delivered from plastic syringes through stainless steel 26 gauge needles.

The animals were observed for 24 hours and painlessly sacrificed at 24 hours. The 24 individual foci of infiltration were excised, fixed in 10% formalin, embedded in paraffin, and stained and examined by standard histopathological methods.

Similar groups of control rats were injected with (1) bovine serum albumin in saline, (2) normal human serum, and (3) physiological saline, and examined and sacrificed as above, with the excised foci of injection treated as above.

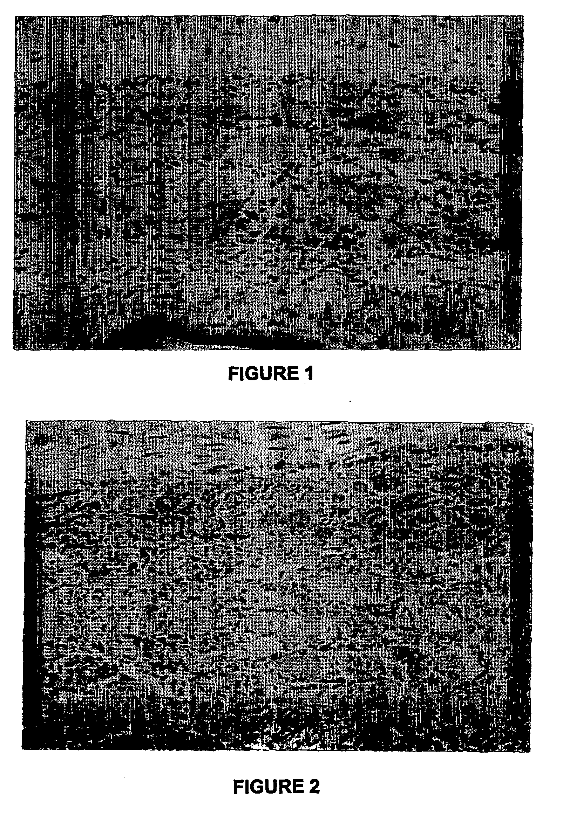

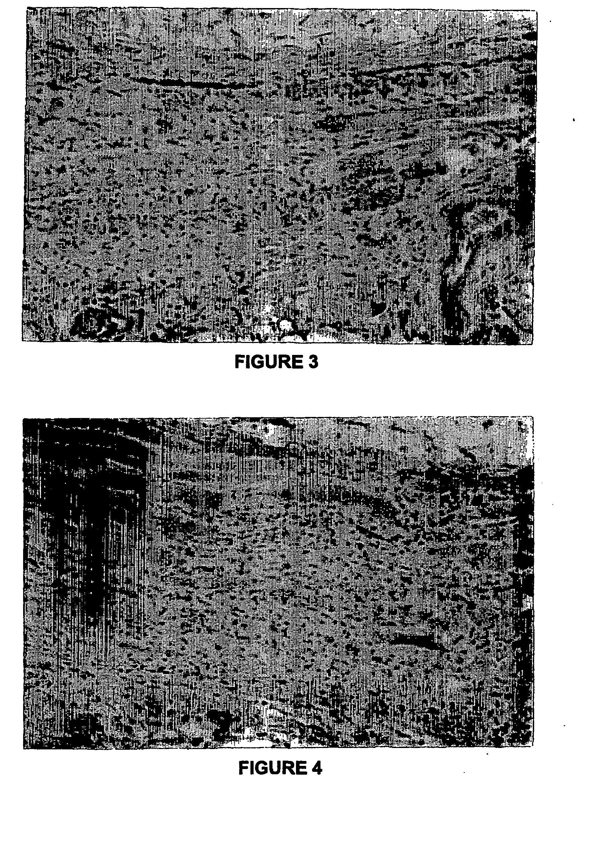

Injection of AD7C-NTP in all examples produced acute necrosis of tissue at the injection sites (Figures 1-4). Figures 1-4 are microphotographs showing the histopathological lesions induced by injection of the AD7C-NTP. The necrosis is evident in muscle tissue, subcutaneous connective tissue, and dermis at the sites where the AD7C-NTP was injected. At 24 hours, cells appear pale, shrunken, and necrotic, and there is infiltration with inflammatory cells. The necrosis correlates with the areas of injection and does not appear to spread far beyond the site of injection.

Controls showed no evidence of necrosis or cell loss. Control injections had mild to minimal acute inflammation and focal microhemorrhages from the needles.

### Example 2

This example demonstrates preventing and/or inhibiting necrosis of live tissue *(in vivo)* by administering an antibody to tissue that contains AD7C-NTP.

The antibody to AD7C-NTP consisted of monoclonal antibody N314 as described in de la Monte, SM, et al., Journal of Neuropathology and Experimental Neurology; 55: 1038-1050 (1996).

AD7C-NTP was obtained as described above in Example 1.

Recombinant AD7C-NTP samples 100 ng/mL-10 µg/mL were incubated at room temperature for 5 minutes to one hour with N314 antibody and then injected into rats as described in Example 1.

The animals were observed for 24 hours and painlessly sacrificed at 24 hours. The 24 individual foci of infiltration were excised, fixed in 10% formalin, embedded in paraffin, and stained and examined by standard histopathological methods.

Similar groups of control rats were injected with (1) AD7C-NTP alone as described in Example 1, (2) bovine serum albumin in saline, (3) normal human serum, and (4) physiological saline, and examined and sacrificed as above, with the excised foci of injection treated as above.

The control injections of AD7C-NTP alone produced tissue necrosis, as described in Example 1, and shown in Figures 1-4. Control injections of bovine serum albumin (BSA), normal human serum, and physiological saline all showed no evidence of necrosis or cell loss. The above control injections had mild to minimal acute inflammation and focal microhemorrhages from the needles.

The AD7C-NTP samples that were injected together with the N314 antibody showed over 95% reduction in tissue necrosis, when compared to the control samples injected with AD7C-NTP alone. There were occasional focal nodules of inflammatory cell foci with micronodule formation which appeared to possibly be aggregation of AD7C-NTP and N314. The overall tissue injury was reduced by >95% by administration of the N314 antibody, when compared to controls that were injected only with AD7C-NTP.

## Claims

1. Use of at least one antibody, antibody fragment or antibody derivative that recognises NTP for the manufacture of an agent for use in prevention, control, amelioration and/or inhibition of cell death and/or tissue necrosis in live tissue containing NTP by contacting said antibody, antibody fragment or antibody derivative with the live tissue containing NTP.

2. The use as claimed in claim 1 of at least one antibody fragment.

3. Use as claimed in claim 1 of at least one antibody derivative.

4. The use as claimed in claim 1 of at least one antibody.

5. Use of at least one antibody, antibody fragment or antibody derivative that recognises NTP for the manufacture of an agent for use in the treatment of conditions caused by cell death and/or tissue necrosis due to the presence of NTP by contacting the antibody, antibody fragment or antibody derivative with the live tissue containing NTP.

6. The use as claimed in claim 5 of at least one antibody fragment.

7. The use as claimed in claim 5 of at least one antibody derivative.

8. The use as claimed in claim 5 of at least one antibody.

9. A composition for treating conditions caused by cell death/or tissue necrosis due to the presence of NTP comprising at least one member selected from the group consisting of an antibody, antibody derivative and antibody fragment that binds NTP, and a component that enables an antibody fragment to cross the blood-brain barrier.

10. The composition of claim 9 for use in preventing and/or inhibiting cell/death and/or tissue necrosis in live mammalian brain tissue containing NTP.

11. The composition of claim 9 for use in treating cerebral amyloidosis.

12. Use of at least one antibody, antibody fragment or antibody derivative that recognises NTP in the manufacture of a medicament for treatment of glaucoma wherein said antibody, antibody fragment or antibody derivative is present in said medicament in sufficient amounts to prevent, control, ameliorate and/or inhibit the cell death and/or tissue necrosis caused by the presence of NTP.

13. The use of at least one member selected from a group consisting of an antibody, antibody derivative and antibody fragment that recognizes NTP, for the manufacture of a medicament for the prevention, modulation, control, amelioration and/or inhibition of cell death and/or tissue necrosis in live tissue at or near the site where NTP has been administered for the purpose of removing or destroying unwanted or harmful cells or tissue, by contacting the composition with the live tissue containing NTP.

## Patentansprüche

1. Verwendung von wenigstens einem Antikörper, Antikörperfragment oder Antikörperderivat, der/das NTP erkennt, zur Herstellung eines Mittels zur Verwendung bei der Vorbeugung, Kontrolle, Verbesserung und/oder Hemmung von Zelltod und/oder Gewebenekrose im NTP enthaltenden lebenden Gewebe durch in Kontakt bringen des Antikörpers, Antikörperfragments oder Antikörperderivats mit dem NTP enthaltenden lebenden Gewebe.

2. Verwendung nach Anspruch 1 von wenigstens einem Antikörperfragment.

3. Verwendung nach Anspruch 1 von wenigstens einem Antikörperderivat.

4. Verwendung nach Anspruch 1 von wenigstens einem Antikörper.

5. Verwendung von wenigstens einem Antikörper, Antikörperfragment oder Antikörperderivat, der/das NTP erkennt, zur Herstellung eines Mittels zur Verwendung bei der Behandlung von Zuständen, die durch Zelltod und/oder Gewebenekrose aufgrund des Vorliegens von NTP verursacht werden, durch in Kontakt bringen des Antikörpers, Antikörperfragments oder Antikörperderivats mit dem NTP enthaltenden lebenden Gewebe.

6. Verwendung nach Anspruch 5 von wenigstens einem Antikörperfragment.

7. Verwendung nach Anspruch 5 von wenigstens einem Antikörperderivat.

8. Verwendung nach Anspruch 5 von wenigstens einem Antikörper.

9. Zusammensetzung zur Behandlung von Zuständen, die durch Zelltod und/oder Gewebenekrose aufgrund des Vorliegens von NTP verursacht werden, umfassend wenigstens ein Element ausgewählt aus der Gruppe bestehend aus einem Antikörper, Antikörperderivat und Antikörperfragment, der/das NTP bindet und einem Bestandteil, der es einem Antikörperfragment ermöglicht, die Bluthirnschranke zu überqueren.

10. Zusammensetzung nach Anspruch 9 zur Verwendung bei der Vorbeugung und/oder Hemmung von Zelltod und/oder Gewebenekrose im lebenden NTP enthaltenden Säugetiergehirngewebe.

11. Zusammensetzung nach Anspruch 9 zur Verwendung bei der Behandlung zerebraler Amyloidose.

12. Verwendung von wenigstens einem Antikörper, Antikörperfragment oder Antikörperderivat, der/das NTP erkennt, zur Herstellung eines Arzneimittels für die Behandlung von Glaukom, wobei der Antikörper, das Antikörperfragment oder das Antikörperderivat in dem Arzneimittel in ausreichenden Mengen vorliegt, um den durch das Vorliegen von NTP verursachte Zelltod und/oder eine Gewebenekrose zu vermeiden, kontrollieren, verbessern und/oder hemmen.

13. Verwendung von wenigstens einem Element ausgewählt aus einer Gruppe bestehend aus einem Antikörper, Antikörperderivat und Antikörperfragment, der/das NTP erkennt, zur Herstellung eines Arzneimittels für die Vorbeugung, Modulation, Kontrolle, Verbesserung und/oder Hemmung von Zelltod und/oder Gewebenekrose im lebenden Gewebe an oder in der Nähe der Stelle, an der NTP zum Zwecke der Entfernung oder Zerstörens unerwünschter oder schädigender Zellen oder Gewebes verabreicht worden ist, durch in Kontakt bringen der Zusammensetzung mit dem NTP enthaltenden lebenden Gewebe.

## Revendications

1. Utilisation d'au moins un anticorps, fragment d'anticorps ou dérivé d'anticorps qui reconnaît les NTP pour la production d'un agent destiné à être utilisé dans la prévention, ou la lutte contre, l'amélioration et/ou l'inhibition de la mort cellulaire et/ou de la nécrose tissulaire dans un tissu vivant contenant des NTP en mettant en contact ledit anticorps, fragment d'anticorps ou dérivé d'anticorps avec le tissu vivant contenant des NTP.

2. Utilisation suivant la revendication 1 d'au moins un fragment d'anticorps.

3. Utilisation suivant la revendication 1 d'au moins un dérivé d'anticorps.

4. Utilisation suivant la revendication 1 d'au moins un anticorps.

5. Utilisation d'au moins un anticorps, fragment d'anticorps ou dérivé d'anticorps qui reconnaît les NTP pour la production d'un agent destiné à être utilisé dans le traitement d'affections provoquées par la mort cellulaire et/ou la nécrose tissulaire due à la présence de NTP en mettant en contact l'anticorps, le fragment d'anticorps ou le dérivé d'anticorps avec le tissu vivant contenant des NTP.

6. Utilisation suivant la revendication 5 d'au moins un fragment d'anticorps.

7. Utilisation suivant la revendication 5 d'au moins un dérivé d'anticorps.

8. Utilisation suivant la revendication 5 d'au moins un anticorps.

9. Composition pour le traitement d'affections provoquées par la mort cellulaire et/ou la nécrose tissulaire due à la présence de NTP, comprenant au moins un membre choisi dans le groupe consistant en un anticorps, un dérivé d'anticorps et un fragment d'anticorps qui se lie aux NTP, et un constituant qui permet à un fragment d'anticorps de traverser la barrière hémato-encéphalique.

10. Composition suivant la revendication 9, destinée à être utilisée dans la prévention et/ou l'inhibition de la mort cellulaire et/ou de la nécrose tissulaire dans un tissu cérébral vivant de mammifère, contenant des NTP.

11. Composition suivant la revendication 9, destinée à être utilisée dans le traitement de l'amyloïdose cérébrale.

12. Utilisation d'au moins un anticorps, fragment d'anticorps ou dérivé d'anticorps qui reconnaît les NTP dans la production d'un médicament destiné au traitement du glaucome, dans laquelle ledit anticorps, fragment d'anticorps ou dérivé d'anticorps est présent dans ledit médicament en des quantités suffisantes pour prévenir, lutter contre, améliorer et/ou inhiber la mort cellulaire et/ou la nécrose tissulaire provoquée par la présence de NTP.

13. Utilisation d'au moins un membre choisi dans un groupe consistant en un anticorps, un dérivé d'anticorps et un fragment d'anticorps reconnaissant les NTP, pour la production d'un médicament destiné à la prévention, la modulation, la lutte contre, l'amélioration et/ou l'inhibition de la mort cellulaire et/ou de la nécrose tissulaire dans un tissu vivant au ou à proximité du site où une NTP a été administrée afin d'éliminer ou détruire des cellules ou un tissu indésirables ou néfastes, en mettant en contact la composition avec le tissu vivant contenant la NTP.
